# EUROPEAN PATENT APPLICATION

(11) **EP 2 301 356 A2**
(43) Date of publication of application: **30.03.2011**
(21) Application number: 10011505.4
(22) Date of filing: 04.12.2003
(51) Int. Cl.: A01N 63/00, A61K 45/00, A61K 47/00, C12N 5/00, C12N 5/02, C12N 5/0784

(54) **Rapid one-step method for generation of antigen loaded dendritic cell vaccine from precursors**

(30) Priority: 04.12.2002 US 430791 P
(62) Divisional of application: 03808435.6
(71) Applicant: Baylor Research Institute, Dallas, TX 75204 (US)
(72) Inventor: Banchereau, Jaques, F., Dallas, TX 75230 (US); Palucka, Anna, Karolina, Dallas, TX 75204 (DE)
(74) Representative: UEXKÜLL & STOLBERG

(57) **Abstract**

The invention relates to antigen loaded antigen-presenting cells made by contacting monocytes *ex vivo* with soluble or particulate antigenic material, tumor necrosis factor alpha, and granulocyte-macrophage colony stimulating factor to form antigen loaded antigen-presenting cells, wherein said antigen-presenting cells comprise one or more subsets selected from the group consisting of Langerhans cells with surface markers (CD1a+ CD14- CD207+); interstitial dendritic cells with surface markers (CD1a+ CD14+ CD207-); double-negative dendritic cells (DN-DCs) with surface markers (CD1a- CD14-); and dendritic cells with surface markers (CD1a- CD14+ CD209+). Said antigen-presenting cells can be used directly as a vaccine or for producing cytotoxic cells that are used for stimulating immune response in a patient.

## Description

### CROSS REFERENCE TO RELATED APPLICATION

This application claims the benefit of U.S. provisional application No. 601430,791 filed December 4, 2002.

### TECHNICAL FIELD OF INVENTION

This invention relates to compositions and methods for rapidly producing antigen loaded dendritic cells from precursors, particularly from monocytes and soluble or particulate antigen(s), and more particularly from killed tumor cells.

### BACKGROUND

The concept of cancer immunotherapy has been energized in the past decade by the molecular identification of human cancer antigens. These therapeutic approaches were further facilitated through identification of in vitro culture methods allowing generation of large numbers of dendritic cells (DCs) on which the cancer antigens can be presented to T cells. Numerous studies in animals have demonstrated that immunization with tumor antigens delivered on DCs induce protective anti-tumor responses. Pioneering clinical trials have used blood derived DCs or monocyte derived DCs and showed some clinical and, more recently, also immune responses. (Bell, et al. 1999. "Dendritic cells," Adv Immunol 72:255; Boczkowski, et al. 1996. "Dendritic cells pulsed with RNA are potent antigen-presenting cells in vitro and in vivo," J Exp Med 184:465-472; Celluzzi, et al. 1996. "Peptide-pulsed dendritic cells induce antigen-specific CTL-mediated protective tumor immunity," JExp Med 183:283-287; Flamand, et al. 1994 "Murine dendritic cells pulsed in vitro with tumor antigen induce tumor resistance in vivo," Eur J Immunol 24:605-610; Gilboa, et al. 1998. "Immunotherapy of cancer with dendritic-cell-based vaccines," Cancer Immunol Immunother 46:82; Gilboa, E. 1999. "The makings of a tumor rejection antigen," Immunity 11: 263-270; Hsu, et al. 1996. "Vaccination of patients with B-cell lymphoma using autologous antigen-pulsed dendritic cells," Nat Med 2:52; Mayordomo, et al. 1995. "Bone marrow-derived dendritic cells pulsed with synthetic tumour peptides elicit protective and therapeutic antitumour immunity," Nat Med 1:1297-1302; Mayordomo, et al. 1997. "Bone marrow-derived dendritic cells serve as potent adjuvants for peptide-based antitumor vaccines," Stem Cells 15:94-103; Mukherji, et al. 1995. "Induction of antigen-specific cytolytic T cells in situ in human melanoma by immunization with synthetic peptide-pulsed autologous antigen presenting cells," Proc Natl Acad Sci USA 92:8078; Nestle, et al. 1998. "Vaccination of melanoma patients with peptide- or tumor lysate-pulsed dendritic cells," Nat Med 4:328; Porgador, A. and Gilboa, E. 1995. "Bone marrow-generated dendritic cells pulsed with a class I-restricted peptide are potent inducers of cytotoxic T lymphocytes," J Exp Med 182:255-260; Song, et al. 1997. "Dendritic cells genetically modified with an adenovirus vector encoding the cDNA for a model antigen induce protective and therapeutic antitumor immunity," JExp Med 186:1247-1256; Song, J. A. 1998. "Tumor immunology: the glass is half full," Immunity 9:757-763; Specht, et al. 1997. "Dendritic cells retrovirally transduced with a model antigen gene are therapeutically effective against established pulmonary metastases," J Exp Med 186:1213-1221; Tjoa, et al. 1998. "Evaluation of phase I/II clinical trials in prostate cancer with dendritic cells and PSMA peptides," Prostate 36:39-44; Toes, et al. 1996. "Protective antitumor immunity induced by immunization with completely allogeneic tumor cells," Cancer Res 56:3782-3787; Zitvogel, et al. 1996. "Therapy of murine tumors with tumor peptide-pulsed dendritic cells: dependence on T cells, B7 costimulation, and T helper cell 1-associated cytokines," J Exp Med 183:87-97; Schuler-Thurner, et al. 2000. "Mage-3 and influenza-matrix peptide-specific cytotoxic T cells are inducible in terminal stage HLA-A2.1+ melanoma patients by mature monocyte-derived dendritic cells," J Immunol 165:3492-3496*;* Mackensen, et al. 2000. "Phase I study in melanoma patients of a vaccine with peptide-pulsed dendritic cells generated in vitro from CD34(+) hematopoietic progenitor cells," Int J Cancer 86:385-392; Geiger, et al. 2001. "Vaccination of pediatric solid tumor patients with tumor lysate-pulsed dendritic cells can expand specific T cells and mediate tumor regression," Cancer Res 61:8513-8519; Heiser, et al. 2002. "Autologous dendritic cells transfected with prostate-specific antigen RNA stimulate CTL responses against metastatic prostate tumors," J Clin Invest 109:409-417; Lodge, et al. 2000. "Dendritic cell-based immunotherapy of prostate cancer: immune monitoring of a phase II clinical trial," Cancer Res 60:829-833; Fong, et al. 2001. "Dendritic cells injected via different routes induce immunity in cancer patients," J Immunol 166:4254-4259; and Banchereau, et al. 2002. "Dendritic cells as vectors for therapy," Cell 106:271-274). While these results are promising, several parameters of DC vaccination need to be established including among others the type of DCs.

As of today, DCs are generated in long term cultures either from hematopoietic progenitors or from blood precursors, i.e., monocytes. (Caux, et al. 1992. "GM-CSF and TNF-alpha cooperate in the generation of dendritic Langerhans cells," Nature 360:258-261; Romani, et al. 1994. "Proliferating dendritic cell progenitors in human blood," J Exp Med 180:83-93; Sallusto, F. and Lanzavecchia, A. 1994. "Efficient presentation of soluble antigen by cultured human dendritic cells is maintained by granulocyte/macrophage colony-stimulating factor plus interleukin 4 and downregulated by tumor necrosis factor alpha," J Exp Med 179:1109-1118; Reid, et al. 1992. "Interaction of tumor necrosis factor with granulocyte-macrophage colony-stimulating factor and other cytokines in the regulation of dendritic cell growth in vitro form bipotent CD34+ progenitors in human bone marrow," J Immunol 149:2681-2688; Arrighi, et al. 1999. "Long-term culture of human CD34(+) progenitors with FLT3-ligand, thrombopoietin, and stem cell factor induces extensive amplification of a CD34(-)CD14(-) and a CD34(-)CD14(+) dendritic cell precursor," Blood 93:2244-2252; Caux, et al. 1990. "Tumor necrosis factor α strongly potentiates interleukin-3 and granulocyte-macrophage colony-stimulating factor-induced proliferation of human CD34+ hematopoietic progenitor cells," Blood 75:2292-2298; Chang, et al. 2000. "Monocyte-derived CD1a+ and CD1a- dendritic cell subsets differ in their cytokine production profiles, susceptibilities to transfection, and capacities to direct Th cell differentiation," Immunology 165:3584-3591; and Weissman, et al. 2000. "HIV gag mRNA transfection of dendritic cells (DC) delivers encoded antigen to MHC class I and II molecules, causes DC maturation, and induces a potent human in vitro primary immune response," Immunology 165:4710-4717).

CD34⁺hematopoietic progenitor cells (HPCs) yield DC preparations upon culture with GM-CSF plus TNF (GM-CSF/TNF) that are composed of two distinct DC subsets, Langerhans cells (LC) and interstitial DCs (intDCs). This contrasts with DCs generated by culturing monocytes with GM-CSF plus IL-4 (GM-CSF/IL-4), which contain a uniform population of immature DCs resembling intDCs. While GM-CSF/IL-4 induced DCs require additional maturation factors, CD34-DCs do not as they are generated in the presence ofTNF alpha, a DC activation factor. U.S. Patent No. 6,004,807 teaches generation of dendritic cells from CD34 hematopoietic progenitors using tissue culture medium supplemented with 10%(v/v) heat inactivated fetal bovine serum.

*Two DC subsets with distinct biological_functions emerge from cultures of cord blood CD34⁺HPCs*: Early studies were performed with cord blood CD34⁺HPC and skewed their differentiation into DCs upon culture with GM-CSF/TNF or IL-3/TNF. In these culture conditions, the cooperation between TNF-alpha and GM-CSF/IL-3 is critical for the development of DCs from CD34⁺ HPC. Indeed, while early studies have suggested TNF as an inhibitor of hematopoiesis, more recently TNF was observed to be a stimulator of the proliferation of CD34⁺ HPC induced by either IL-3 or GM-CSF. Limiting dilution analysis indicated that TNF increases both the frequency of IL-3 responding cells and the average size of the IL-3-dependent clones. After 12 days of liquid culture, CD34⁺ HPC cultured with GM-CSF and TNF yield both adherent and non-adherent cells with a dendritic morphology. For optimal generation of DCs, TNF is mostly required during the first days of the culture where it upregulates the expression of the common chain subunit of the GM-CSF/IL-3/IL-5 receptor. Another important site of action of TNF in the CD34⁺ HPC cultures is the inhibition of granulopoietic differentiation and proliferation. In particular, TNF reversibly blocks granulocytic differentiation at the level of uncommitted CD13⁻CD15⁻ blast cells that accumulate in TNF enriched cultures. Furthermore, growth of committed granulocytic progenitors (CD15⁺) is inhibited through an arrest of cell cycle in G0/G1 (Caux, et al. 1991. "Potentiation of early hematopoiesis by tumor necrosis factor-alpha is followed by inhibition of granulopoietic differentiation and proliferation," Blood 78:635-644; Jacobsen, et al. 1992. "Tumor necrosis factor alpha directly and indirectly regulates hematopoietic progenitor cell proliferation: role of colony-stimulating factor receptor modulation," JExp Med 175:1759-1772). TNF also blocks erythropoiesis in these cultures. These effects of TNF on growing CD34⁺ HPC are mediated through the p55 TNF Receptor (Rusten, et al. 1994. "Bifunctional effects of tumor necrosis factor alpha (TNF alpha) on the growth of mature and primitive human hematopoietic progenitor cells: involvement of p55 and p75 TNF receptors," Blood 83:3152-3159).

As mentioned above, two precursor subpopulations emerge under these culture conditions, CD1a⁺ and CD14⁺, which can differentiate into LC and intDC, respectively (Caux, et al. 1996. "CD34+ hematopoietic progenitors from human cord blood differentiate along two independent dendritic cell pathways in response to GM-CSF+TNF alpha," J Exp Med 184:695-706; Caux, et al. 1997. "CD34+ hematopoietic progenitors from human cord blood differentiate along two independent dendritic cell pathways in response to granulocyte-macrophage colony-stimulating factor plus tumor necrosis factor alpha: II. Functional analysis," Blood 90:1458-1470). Both precursor subsets mature at Day 12-14 into DCs with typical morphology, phenotype (CD80, CD83, CD86, CD58, high HLA class II) and function. CD1a⁺ precursors give rise to cells with Langerhans cell characteristics (Birbeck granules, Lag antigen and E-cadherin). In contrast, the CD14⁺ precursors mature into CD1a⁺ DC lacking Birbeck granules, E-cadherin and Lag antigen but expressing CD2, CD9, CD68 and the coagulation factor XIIIa described in dermal dendritic cells. Interestingly, the CD14⁺ precursors, but not the CD1a⁺ precursors, represent bipotent cells that can be induced to differentiate, in response to M-CSF, into macrophage-like cells, lacking accessory function for T cells. These DC subsets differ with regard to: (1) antigen capture; intDC are more efficient in Dextran uptake and in binding immune complexes than LC; (2) lysosomal activity, with intDC expressing higher levels of enzymatic activity; (3) capacity to induce naive B cell differentiation, with intDC uniquely able to induce the priming of naïve B cells and their differentiation into IgM secreting plasma cells through the secretion of IL-12 (Dubois, et al. 1997. "Dendritic cells enhance growth and differentiation of CD40-activated B lymphocytes," JExp Med 185:941-951; Dubois, et al. 1998. "Critical role ofIL-12 in dendritic cell-induced differentiation of naive B lymphocytes," J Immunol 161:2223-2231); and (4) while both subsets express IL-12 upon CD40 ligation, only intDC express IL-10. While the unique function of LCs remains to be established, there are some reports suggesting that CD34-DCs with LC component are more efficient in CD8 T cell priming than monocyte-derived DCs (Mortarini, et al. 1997. "Autologous dendritic cells derived from CD34+ progenitors and from monocytes are not functionally equivalent antigen-presenting cells in the induction of melan-A/Mart-1(27-35)-specific CTLs from peripheral blood lymphocytes of melanoma patients with low frequency of CTL precursors," Cancer Res 57:5534-5541; and Ferlazzo, et al. 2000. "Dendritic cells generated from CD34+ progenitor cells with flt3 ligand, c-kit ligand, GM-CSF, IL-4, and TNF-alpha are functional antigen-presenting cells resembling mature monocyte-derived dendritic cells," J Immunother 23:48-58.). These observation are confirmed in our own studies demonstrating that DCs with Langerhans cells phenotype induced by culturing monocytes with GM-CSF and In- 15 are more efficient in induction of CTL responses in vitro than GM-CSF/IL-4 DCs (Mohamadzadeh et al. 2001. "Interleukin 15 skews monocyte differentiation into dendritic cells with features of Langerhans cells," J Exp Med 194:1013-20). In the circumstances like the induction of tumor-specific CTLs, CD34-DCs or any other DC preparation that contains Langerhan cells could thus be advantageous.

*G-CSF* mobilized *blood CD34⁺HPC also yield two DC subsets:* Peripheral blood mobilized by G-CSF and isolated from patients with advanced melanoma follow the differentiation pathway recognized for cord blood, and at Day 9 of culture, two populations can be identified, i.e., CD19⁺CD14⁻ LCs and CD1a⁻CD14⁺ intDCs. About 50% of cells in these cultures remain, however, at the progenitor/precursor stage and can give rise to DCs upon further culture. Thus, these cultures are asynchronic, a parameter that needs to be taken into consideration when using these DC preparations as vaccine. Both subsets are CD83^{low} CD86⁺ HLA-DR⁺, MHC class I⁺ (intDC express slightly higher levels of class I), LC express higher levels of CD11c and CD80. Their Langerhans cell phenotype is confirmed by confocal microscopy revealing Lag expression and cytoplasmic structures corresponding to Birbeck granules, both found exclusively in LCs. LCs and intDCs sorted from Day 9 cultures display DC properties as determined by the following: 1) morphology, wherein Giemsa staining reveals distinct nuclear and cytoplasmic structure with prominent dendrites; 2) antigen capture, wherein intDC capture considerably more FITC-Dextran than LCs; and 3) induction of T cell responses, wherein while both CD1a⁺CD14⁻ and CD1a⁺ CD14⁺ cells can induce allogeneic T cell proliferation, sorted CD1a⁺ cells are much more efficient than CD14⁺ cells in inducing recall responses to TT (using autologous CD4 T cells) as well as the proliferation of allogeneic CD8 T cells. LCs and intDCs, induced to maturation by CD40L, IFN alpha or synthetic dsRNA (poly I:C), give comparably increased proliferative T cell responses.

### Antigen-loaded CD34⁺HPCs-derived DCs elicit immune and clinical responses:

As disclosed in International Application No. PCT/US02/07232 (WO 02/072013), eighteen HLA A*0201⁺ patients with stage IV melanoma were vaccinated with CD34⁺ HPC-derived DCs pulsed with six Ags: influenza matrix peptide (Flu-MP), KLH, and peptides derived from the four melanoma Ags: MART-1/Melan A, gp100, tyrosinase and MAGE-3. The DCs were loaded with KLH, Flu matrix peptide and HLA-A2 binding peptides derived from four melanoma antigens MAGE-3, MART-1/MELAN A, GP-100 and Tyrosinase and administered bi-weekly over 6 weeks (four s.c. injections). As described above, these DCs contain a Langerhans cell component. Vaccination of patients with advanced melanoma with antigen pulsed CD34-DCs proved to be well tolerated and resulted in enhanced immunity to both "non-self ' (viral peptide and KLH protein) and "self" (melanoma peptides) antigens. It was observed that the level of immune responses in the blood correlated with early outcome at the tumor sites, thus providing further stimulus for the idea that the measurement of immune responses in the blood helps evaluate vaccine efficacy. Indeed, antigen-pulsed CD34+ HPC-derived DCs induced primary and recall immune responses detectable directly in the blood. An immune response to control antigens (KLH, Flu-MP) was observed in 16 of 18 patients. An enhanced immune response to 1 or more melanoma antigens (MelAg) was seen in these same 16 patients, including 10 patients who responded to > 2 MelAg. The MelAg-specific T cells elicited after DC vaccine were functional, and detectable in effector T cell assays without the need for prior ex vivo expansion. They were also capable of proliferation and effector function after short-term (1 week) coculture with antigen bearing DCs, without the need for exogenous cytokines or multiple restimulations with antigen. The two patients failing to respond to both control and tumor antigens experienced rapid tumor progression. Of 17 patients with evaluable disease, 6/7 patients with immunity to 2 or less MelAg had progressive disease at 10 weeks after study entry, in contrast to tumor progression in only 1/10 patients with immunity to > 2 MelAg. Regression of > 1 tumor metastases were observed in 7 of these patients. The overall immunity to melanoma antigens following DC vaccination was associated with clinical outcome (p=0.015).

The analysis of blood immune responses demonstrated that Ags-pulsed CD34-DCs could lead to rapid induction of CD4+ and CD8+T cell immunity. Indeed, a single DC vaccination was sufficient for induction of KLH specific responses in 6 patients and Flu-MP-specific responses in 8 patients. A single DC vaccine was sufficient to induce tumor specific effectors to ≥ 1 melanoma antigen in 5 patients. None of these 5 patients showed early disease progression. Only 1/6 patients with rapid KLH-response experienced early disease progression. Rapid and slow Flu-MP responders did not differ with regard to disease progression. Thus, a patient's ability to rapidly respond to CD4 epitopes or to melanoma Ags could be an early indicator of clinical outcome after DC based vaccination.

Eleven patients received 4 additional "boosting" injections. Analysis of overall survival from study entry revealed that 12/18 patients were alive at Year 1 and 9/18 patients were alive at Year 2. Overall survival from study entry was related to the level of vaccine-specific immune responses detected at 10 weeks, i.e., after the initial 4 injections, as measured by (1) the magnitude, i.e. the number of melanoma Ags-specific IFN gamma ELISPOTS, and (2) the breadth, i.e., the number of melanoma antigens to which T cells respond. Finally, long lasting tumor regression was associated with epitope spreading. Thus, Langerhans cell component in a DC vaccine was shown to be beneficial.

Dendritic cells are increasingly used as vectors for vaccination to induce antigen specific immune responses, for example in patients with cancer. In view of the evidence that immunization with tumor antigens delivered on DCs induces protective anti-tumor responses and that vaccination of patients with antigen-loaded DCs can result in increased immune responses against tumor antigens, vaccination with antigen-loaded DCs has been recommended for treatment of tumors and infectious agents. Thus, improvements in the methods for generating antigen-loaded DCs are beneficial for any vaccination protocol. Current methods for generation of DC vaccines ex vivo are based on culturing either CD34+ hematopoietic progenitors (U.S. Patent No. 6,004,807) or monocytes (with GM-CSF and IL-4 followed by a maturation step), which constitute dendritic cell precursors. These cultures are, however, long and require at least 9 and 7 days for progenitors and precursors, respectively. Furthermore, after dendritic cells have been generated the DCs need to be pulsed with an antigen(s), which requires additional culture.

A method has been found to rapidly generate antigen-loaded dendritic cell vaccine within as little as three (3) days. The antigen-loaded dendritic cells generated by this method can be used in any therapeutic, diagnostic, prophylactic or research protocol.

### SUMMARY OF THE INVENTION

In one aspect, the invention is a method for producing antigen loaded antigen-presenting cells from monocytes ex vivo comprising simultaneously contacting the monocytes with soluble or particulate antigenic material, tumor necrosis factor alpha, and granulocyte-macrophage colony stimulating factor. Preferably, the antigen loaded antigen-presenting cells are produced in less than four days. The antigenic material useful in this method consists of one or more materials selected from the group consisting of antigenic peptides, peptide mimetics, proteins, polyproteins, immune complexes, whole dying cell bodies, dying cell body fragments, viral vectors and liposomes. In a preferred method, the antigenic material consists of soluble antigenic material. In another preferred method, the antigenic material consists of whole dying cell bodies, dying cell body fragments or both. In another preferred method, the antigen loaded antigen-presenting cells can be separated into fractions containing one or more subsets selected from the group consisting of cells with surface markers (CD1a+ CD207+); cells with surface markers (CD1a+ CD207-); cells with surface markers (CD1a-CD14-); and cells with surface markers (CD14+ CD1a-CD209+).

In another aspect, the invention is a method for producing antigen loaded antigen-presenting cells from monocytes ex vivo comprising contacting the monocytes with tumor necrosis factor alpha and granulocyte-macrophage colony stimulating factor at one time point to form antigen-presenting cells and contacting the antigen-presenting cells with soluble or particulate antigenic material at a second time point to form antigen loaded antigen-presenting cells, wherein the antigen loaded antigen-presenting cells are produced in less than four days. The antigenic material useful in this method consists of one or more materials selected from the group consisting of antigenic peptides, peptide mimetics, proteins, polyproteins, immune complexes, whole dying cell bodies, dying cell body fragments, viral vectors and liposomes. In a preferred method, the antigenic material consists of soluble antigenic material. In another preferred method, the antigenic material consists of whole dying cell bodies, dying cell body fragments or both. In another preferred method, the antigen loaded antigen-presenting cells can be separated into fractions containing one or more subsets selected from the group consisting of cells with surface markers (CD1a+ CD207+); cells with surface markers (CD1a+ CD207-); cells with surface markers (CD1a-CD14-); and cells with surface markers (CD14+ CD1a- CD209+).

In another aspect, the invention is a vaccine comprising antigen loaded antigen-presenting cells made according to the steps of simultaneously contacting monocytes ex vivo with soluble or particulate antigenic material, tumor necrosis factor alpha, and granulocyte-macrophage colony stimulating factor to form antigen loaded antigen-presenting cells. Preferably, the antigen loaded antigen-presenting cells are produced in less than four days. The antigenic material in this vaccine consists of one or more materials selected from the group consisting of antigenic peptides, peptide mimetics, proteins, polyproteins, immune complexes, whole dying cell bodies, dying cell body fragments, viral vectors and liposomes. In a preferred vaccine, the antigenic material consists of soluble antigenic material. In another preferred vaccine, the antigenic material consists of whole dying cell bodies, dying cell body fragments or both.

In another aspect, the invention is a vaccine comprising antigen loaded antigen-presenting cells made according to the steps of contacting monocytes ex vivo with tumor necrosis factor alpha and granulocyte-macrophage colony stimulating factor at one time point to form antigen-presenting cells and contacting the antigen-presenting cells with soluble or particulate antigenic material at a second time point to form antigen loaded antigen-presenting cells, wherein the antigen loaded antigen-presenting cells are produced in less than four days. The antigenic material in this vaccine consists of one or more materials selected from the group consisting of antigenic peptides, peptide mimetics, proteins, polyproteins, immune complexes, whole dying cell bodies, dying cell body fragments, viral vectors and liposomes. In a preferred vaccine, the antigenic material consists of soluble antigenic material. In another preferred vaccine, the antigenic material consists of whole dying cell bodies, dying cell body fragments or both.

In another aspect, the invention is a vaccine comprising monocyte-derived antigen loaded antigen-presenting cells, wherein the antigen-presenting cells comprise Langerhans cells and interstitial dendritic cells.

In another aspect, the invention is a vaccine comprising monocyte-derived antigen loaded antigen-presenting cells, wherein the antigen-presenting cells consist of two or more subsets selected from the group consisting of cells with surface markers (CD1a+ CD207+); cells with surface markers (CD1a+ CD207-); cells with surface markers (CD1a-CD 14-); and cells with surface markers (CD 14+ CD 1 a- CD209+).

In another aspect, the invention is a method of inducing tumor specific immune response in a tumor-bearing patient comprising (1) producing a vaccine comprising antigen loaded antigen-presenting cells, wherein the antigen loaded antigen-presenting cells are derived from monocytes ex vivo by simultaneously contacting the monocytes with soluble or particulate antigenic material possessing at least one tumor antigen, tumor necrosis factor alpha, and granulocyte-macrophage colony stimulating factor; and (2) administering the vaccine to the patient, wherein the patient's cells upon contact with the antigen loaded antigen-presenting cells in the vaccine mature to form cytotoxic cells expressing cytotoxic activity against the tumor cells. In another embodiment, the antigen loaded antigen-presenting cells are made by contacting monocytes ex vivo with tumor necrosis factor alpha and granulocyte-macrophage colony stimulating factor at one time point to form antigen-presenting cells and contacting said antigen-presenting cells with soluble or particulate antigenic material possessing at least one tumor antigen at a second time point to form antigen loaded antigen-presenting cells, wherein said antigen loaded antigen-presenting cells are produced in less than four days. In a preferred method, the antigenic material consists of soluble antigenic material. In another preferred method, the antigenic material consists of whole dying cell bodies, dying cell body fragments or both. In one preferred method, the targeted cytotoxic cells are CD4 positive. In another preferred method, the targeted cytotoxic cells are CD8 positive.

In another aspect, the invention is a method of inducing tumor specific responses in a tumor-bearing patient comprising (1) isolating monocytes and cytotoxic cell precursors from the patient; (2) producing antigen loaded antigen-presenting cells from the patient's monocytes by simultaneously contacting the monocytes with soluble or particulate antigenic material possessing at least one tumor antigen, tumor necrosis factor alpha, and granulocyte-macrophage colony stimulating factor; (3) coculturing the loaded antigen-presenting cells with the isolated cytotoxic cell precursors under conditions for their maturation to form cytotoxic cells; and (4) administering the cytotoxic cells to the patient, whereupon the cytotoxic cells express cytotoxic activity against the tumor cells. In another embodiment, the antigen loaded antigen-presenting cells are made by contacting monocytes ex vivo with tumor necrosis factor alpha and granulocyte-macrophage colony stimulating factor at one time point to form antigen-presenting cells and contacting said antigen-presenting cells with soluble or particulate antigenic material possessing at least one tumor antigen at a second time point to form antigen loaded antigen-presenting cells, wherein said antigen loaded antigen-presenting cells are produced in less than four days. In a preferred method, the antigenic material consists of soluble antigenic material. In another preferred method, the antigenic material consists of whole dying cell bodies, dying cell body fragments or both. In one preferred method, the cytotoxic cells are CD4 positive. In another preferred method, the cytotoxic cells are CD8 positive.

In another aspect, the invention is a method of inducing an immune response in a patient comprising (1) producing a vaccine comprising antigen loaded antigen-presenting cells, wherein the antigen loaded antigen-presenting cells are derived from monocytes ex vivo by simultaneously contacting the monocytes with soluble or particulate antigenic material possessing at least one antigen for which an immune response is desired, tumor necrosis factor alpha, and granulocyte-macrophage colony stimulating factor; and (2) administering the vaccine to the patient, wherein the patient's cells upon contact with the loaded antigen-presenting cells in the vaccine mature to form cells expressing immunologic activity against the antigen. In a preferred embodiment, the antigen loaded antigen-presenting cells are made by contacting monocytes ex vivo with tumor necrosis factor alpha and granulocyte-macrophage colony stimulating factor at one time point to form antigen-presenting cells and contacting said antigen-presenting cells with soluble or particulate antigenic material possessing at least one antigen for which an immune response is desired at a second time point to form antigen loaded antigen-presenting cells, wherein said antigen loaded antigen-presenting cells are produced in less than four days. In a preferred method, the antigenic material consists of soluble antigenic material. In another preferred method, the antigenic material consists of whole dying cell bodies, dying cell body fragments or both. In one preferred method, the targeted cytotoxic cells are CD4 positive. In another preferred method, the targeted cytotoxic cells are CD8 positive.

In another aspect, the invention is a method for mounting an immune response mediated by cytotoxic T cells comprising (1) producing antigen loaded antigen-presenting cells from monocytes ex vivo by simultaneously contacting the monocytes with soluble or particulate antigenic material possessing at least one antigen for which an immune response is desired, tumor necrosis factor alpha, and granulocyte-macrophage colony stimulating factor; (2) coculturing the loaded antigen-presenting cells with naïve T cells under conditions for T cell maturation to form cytotoxic T cells; and (3) contacting the cytotoxic T cells with target cells possessing the antigen, wherein the cytotoxic T cells express cytotoxic activity against the target cells possessing the antigen. In another embodiment, the antigen loaded antigen-presenting cells are made by contacting monocytes ex vivo with tumor necrosis factor alpha and granulocyte-macrophage colony stimulating factor at one time point to form antigen-presenting cells and contacting said antigen-presenting cells with soluble or particulate antigenic material possessing at least one antigen for which an immune response is desired at a second time point to form antigen loaded antigen-presenting cells, wherein said antigen loaded antigen-presenting cells are produced in less than four days. In a preferred method, the antigenic material consists of soluble antigenic material. In another preferred method, the antigenic material consists of whole dying cell bodies, dying cell body fragments or both. In one preferred method, the cytotoxic cells are CD4 positive. In another preferred method, the cytotoxic cells are CD8 positive.

In another aspect, the invention is a method for modifying an immune response in a patient comprising (1) producing antigen loaded antigen-presenting cells, wherein the antigen loaded antigen-presenting cells are derived from monocytes ex vivo by simultaneously contacting the monocytes with soluble or particulate antigenic material possessing at least one antigen for which an immune response is desired, tumor necrosis factor alpha, and granulocyte-macrophage colony stimulating factor; and (2) administering the antigen-presenting cells to the patient, wherein the patient's cells upon contact with the loaded antigen-presenting cells are modulated not to express immunologic activity against the antigen.

In yet another aspect, the invention is a method for producing an antigen loaded antigen presenting cell fraction composed of two or more subsets comprising (1) simultaneously contacting monocytes ex vivo with soluble or particulate antigenic material, tumor necrosis factor alpha, and granulocyte-macrophage colony stimulating factor to form antigen loaded antigen-presenting cells; (2) isolating from the antigen loaded antigen-presenting cells two or more subsets wherein each subset is capable of eliciting a distinct T cell response and mounting a distinct immune response when administered to a patient; and (3) combining two or more subsets to form the antigen loaded antigen presenting cell fraction.

### BRIEF DESCRIPTION OF THE DRAWINGS

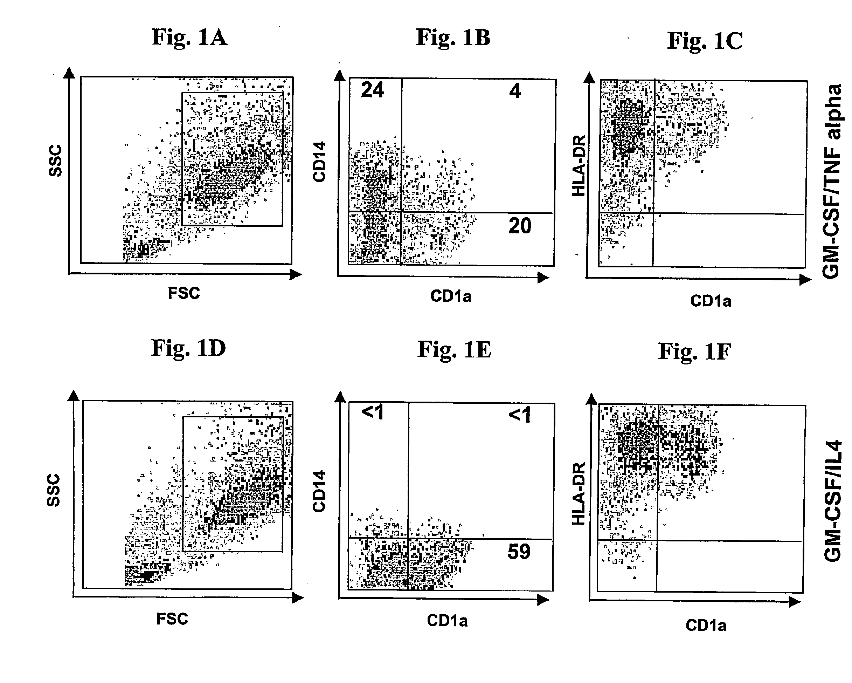

Figs. 1A-F depict the phenotype of Day 3 monocytes cultured with GM-CSF/TNF alpha (Figs. 1A-1C) as compared to monocytes cultured with GM-CSF/IL-4 (Figs. 1D-1F), in terms of FSC/SSC, CD1a/CD14, and CD1a/HLA-DR. As expected in control cultures with GM-CSF and IL-4, the cells became CD 14 negative, and approximately 50% of the cells acquired CD1a expression consistent with DC differentiation (Fig. 1E). However, in Day 3 cultures with GM-CSF and TNF alpha, two separate populations were seen: CD1a+CD14- (20%) cells and CD1a-CD14+ (24%) cells (Fig. 1B).

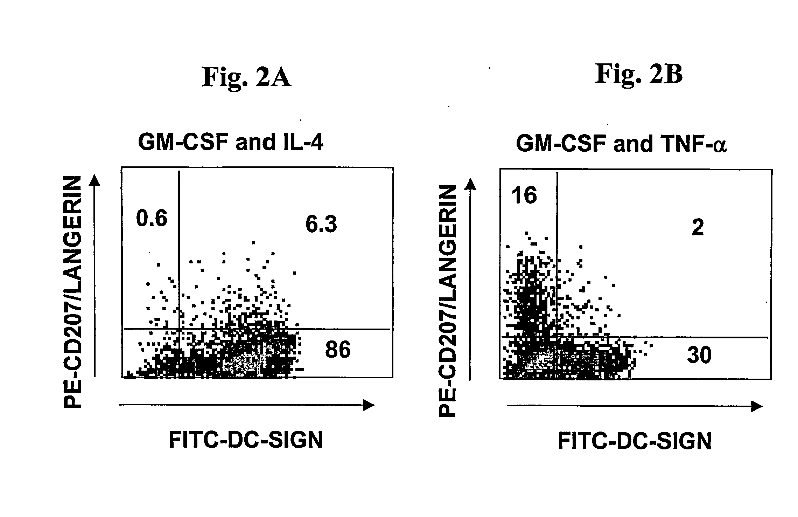

Figs. 2A and 2B depict the phenotype of Day 3 monocytes cultured with GM-CSF/'INF alpha (Fig. 2B) as compared to monocytes cultured with GM-CSF/IL-4 (Fig. 2A), in terms of the expression of DC-SIGN and Langerin, surface markers of interstitial DCs (intDCs) and Langerhans cells (LCs), respectively. TNF cultures contained cells differentially expressing DC-SIGN and Langerin. Contrary to TNF-DCs, IL4-DCs were homogenous, and a majority of cells were DC-SIGN+ Langerin- consistent with intDC differentiation.

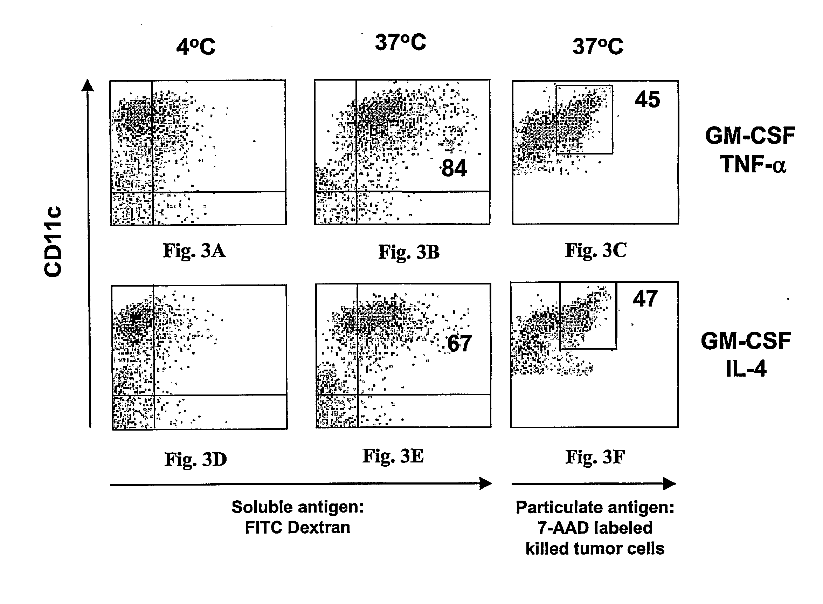

Figs. 3A-3F depict the antigen capture efficiency of dendritic cells generated by culturing monocytes for 3 days with GM-CSF/TNF alpha compared to dendritic cells made by GM-CSF/IL-4 treatment methods. TNF-DCs were as efficient in capturing both soluble (FITC-Dextran) (Figs. 3A-3B) and particulate (killed tumor cells) antigens (Fig. 3C) as GM-CSF/IL-4 DCs (soluble FITC-Dextran in Figs. 3D and 3E; and particulate antigen in Fig. 3F).

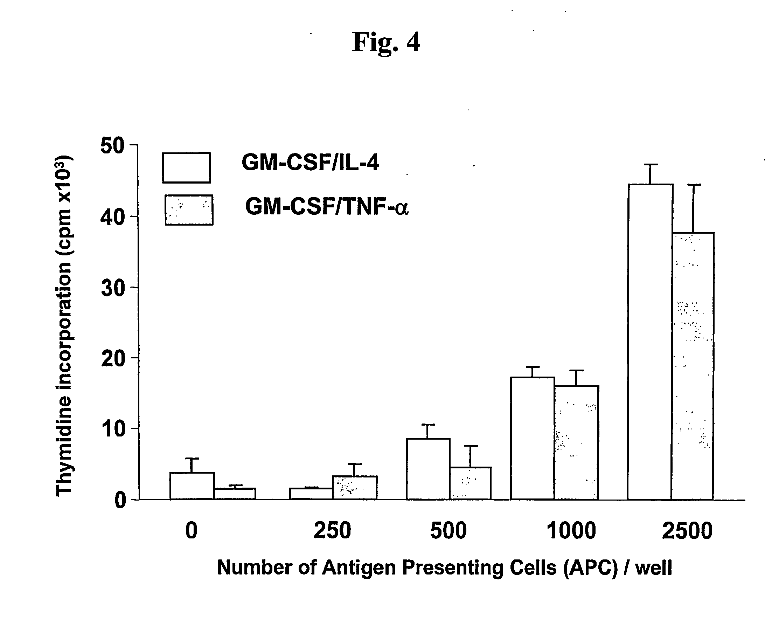

Fig. 4 depicts the ability of Day 3 GM-CSF/TNF DCs to initiate an immune response as demonstrated by the induction of allogeneic CD4 T cell proliferation as compared to GM-CSF/IL-4 DCs. Both Day 3 GM-CSF/TNF DCs and GM-CSF/IL-4 DCs demonstrated the ability to induce CD4 T cell proliferation.

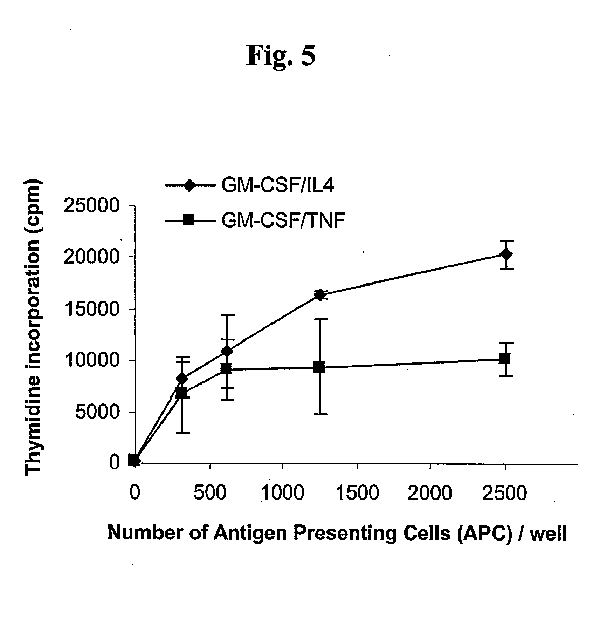

Fig. 5 depicts the ability of Day 3 GM-CSF/TNF DCs to induce allogeneic CD8 T cell proliferation as compared to GM-CSF/IL,-4 DCs. Both Day 3 GM-CSF/TNF DCs and GM-CSF/IL-4 DCs demonstrated the ability to induce CD8 T cell proliferation.

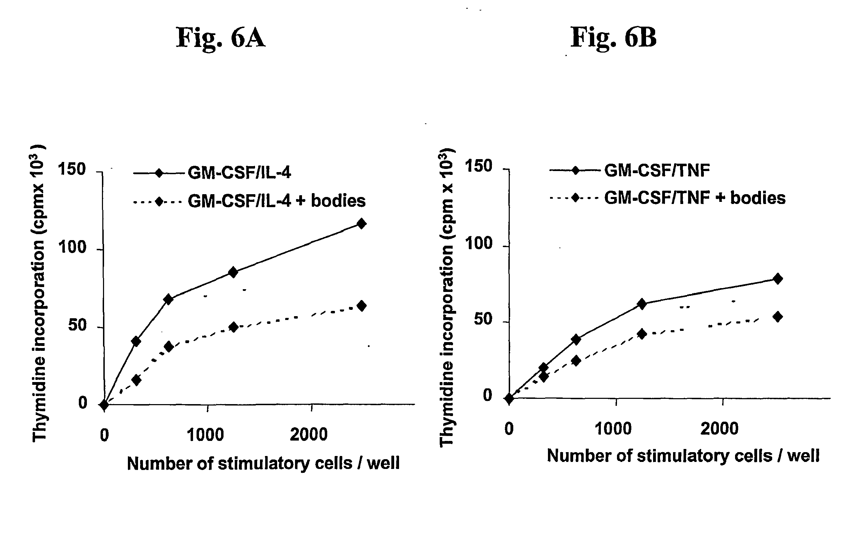

Figs. 6A and 6B depict the capacity of monocytes cultured for 3 days with or without killed tumor cells and either GM-CSF/TNF alpha (Fig. 6B) or GM-CSF/IL-4 (Fig. 6A) to induce allogeneic CD4 T cell proliferation. The one-step vaccine of Day 3 GM-CSF/TNF alpha/killed tumor cells was able to induce allogeneic CD4 T cell proliferation.

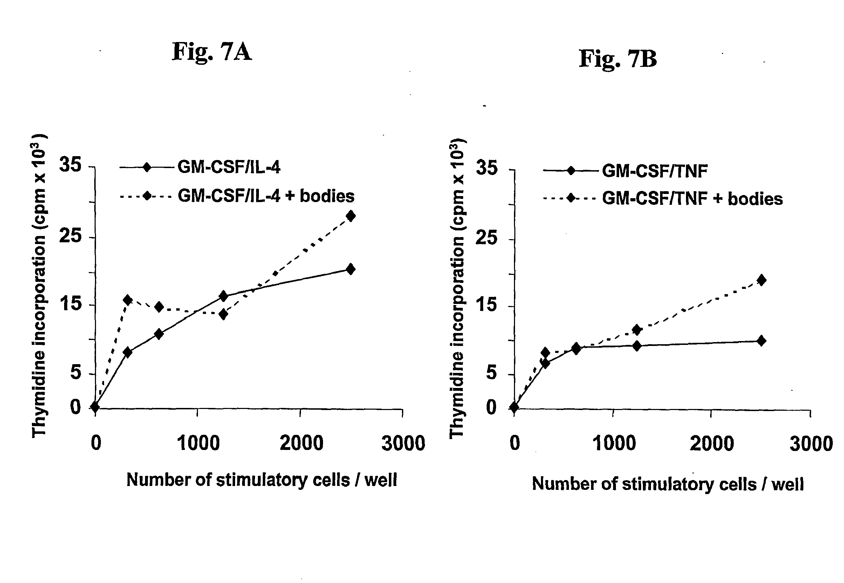

Figs. 7A and 7B depict the capacity of monocytes cultured for 3 days with or without killed tumor cells and either GM-CSF/TNF alpha (Fig. 7B) or GM-CSF/IL-4 (Fig. 7A) to induce allogeneic CD8 T cell proliferation. The one-step vaccine of Day 3 GM-CSF/TNF alpha/killed tumor cells was able to induce allogeneic CD8 T cell proliferation.

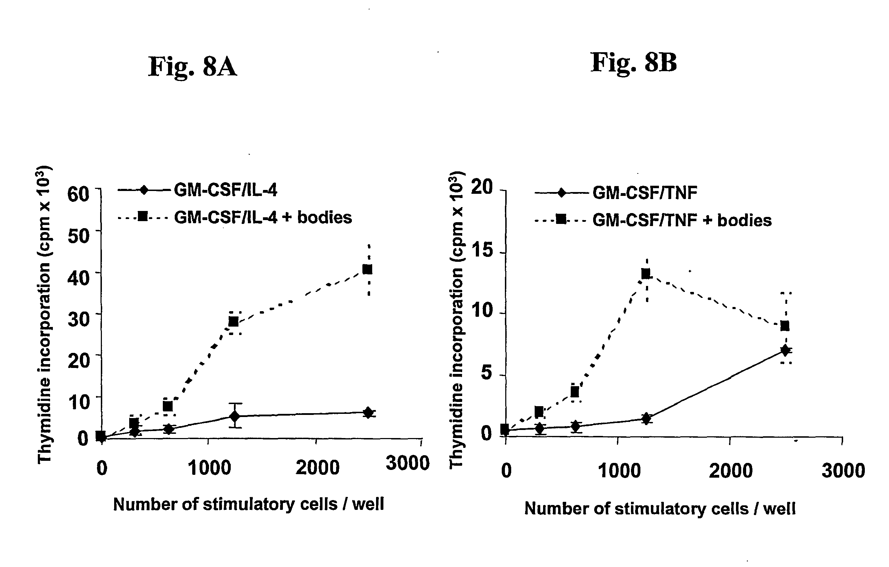

Figs. 8A and 8B depict the capacity of monocytes cultured for 3 days with or without killed tumor cells and either GM-CSF/TNF alpha (Fig. 8B) or GM-CSF/IL-4 (Fig. 8A) to present tumor cell antigens to autologous CD4 T cells. The one-step vaccine of Day 3 GM-CSF/TNF alpha/killed tumor cells was able to present tumor cell antigens to autologous CD4 T cells as demonstrated by their capacity to induce proliferation of autologous CD4 T cells.

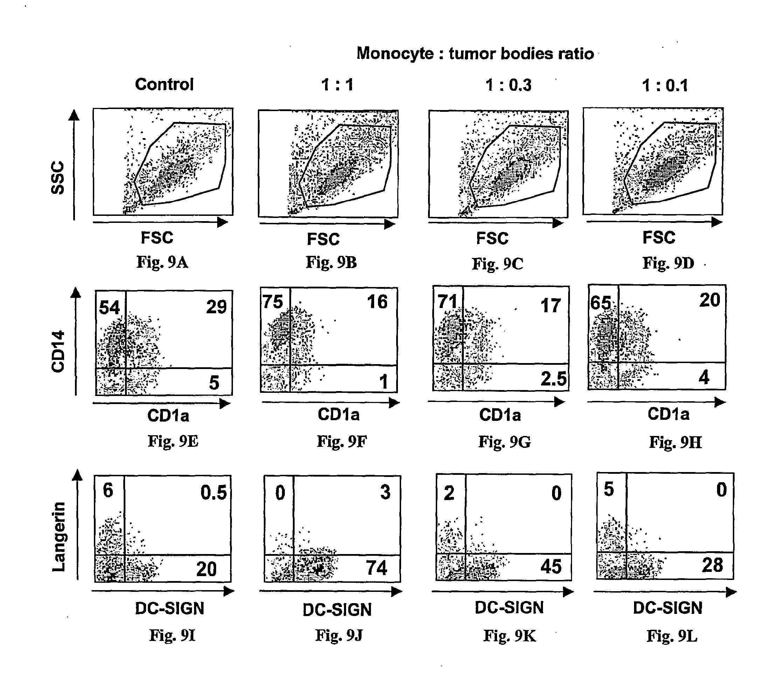

Figs. 9A-9L depict the capacity of monocytes cultured for 3 days with or without killed tumor cells and with GM-CSF/TNF alpha to differentiate into two subsets of dendritic cells, i.e. Langerhans cells (Langerin+ and DC-SIGN-) and interstitial dendritic cells (Langerin- and DC-SIGN+). Titration of tumor bodies shows optimal differentiation at the monocyte:tumor bodies ratio 1:0.1 (Fig. 9L).

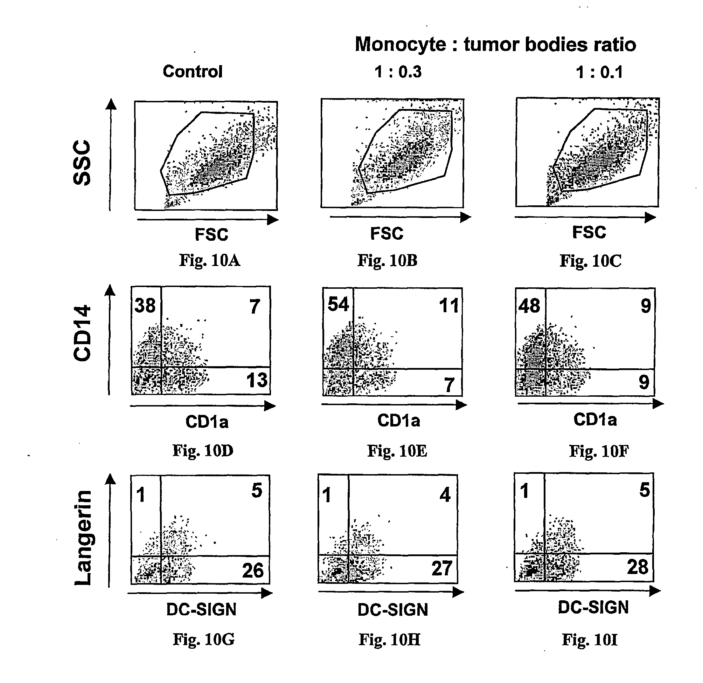

Figs. 10A-10I depict the capacity of monocytes cultured for 3 days with GM-CSF/TNF alpha and with killed tumor cells added at graded doses at Day 2 of monocyte culture to differentiate into two subsets of dendritic cells, i.e. Langerhans cells (Langerin+ and DC-SIGN-) and interstitial dendritic cells (Langerin- and DC-SIGN+).

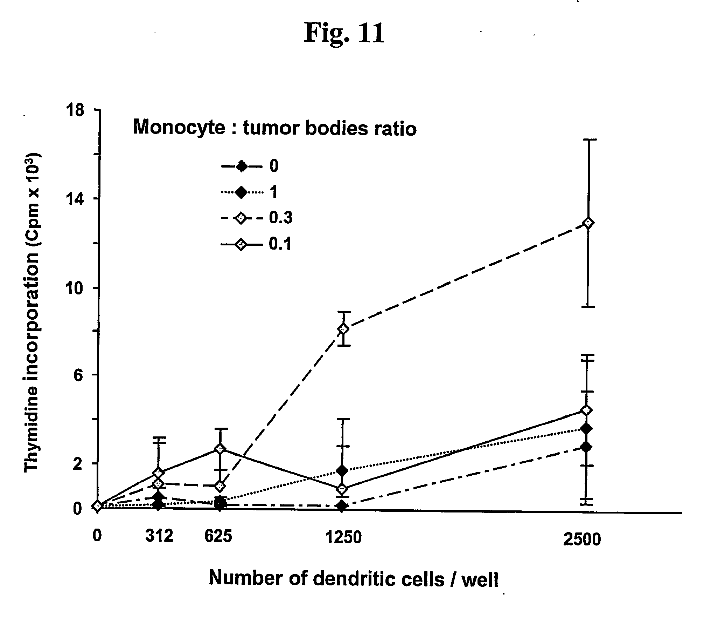

Fig. 11 depicts the capacity of monocytes cultured for 3 days with or without killed tumor cells and with GM-CSF/TNF alpha to present tumor cell antigens to autologous immune effectors (peripheral blood lymphocytes). The optimal presentation is observed at the monocyte:tumor bodies ratio of 1:0.3.

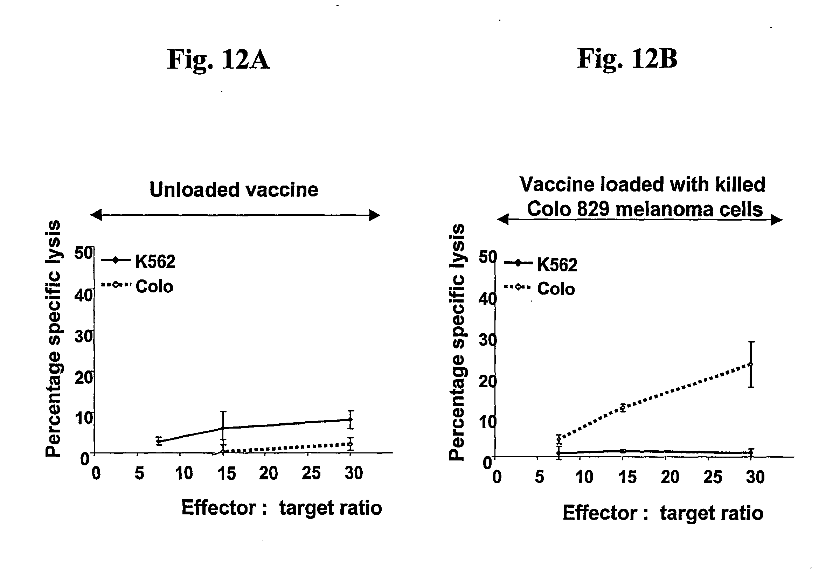

Figs. 12A and 12 B depict the capacity of monocytes cultured for 3 days with (Fig. 12B) or without (Fig. 12A) killed tumor cells and with GM-CSF/TNF alpha to induce differentiation of autologous CD8+ T cells into cytotoxic T cells (CTLs) able to kill melanoma cells already after 2 weeks of culture.

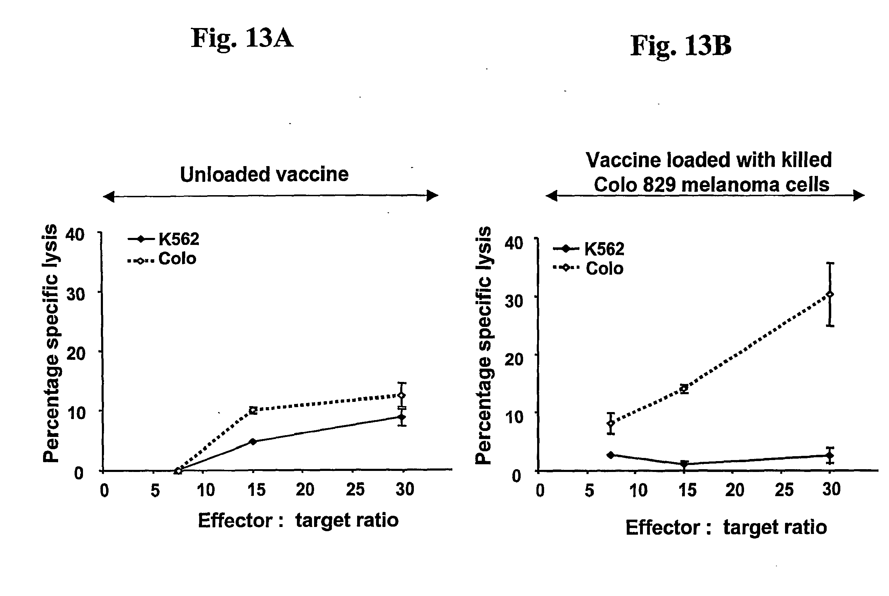

Figs. 13A and 13B depict the capacity of monocytes cultured for 3 days with (Fig. 13B) or without (Fig. 13A) killed tumor cells and with GM-CSF/TNF alpha to induce differentiation of autologous CD8+ T cells into cytotoxic T cells (CTLs) able to kill melanoma cells after 3 stimulations.

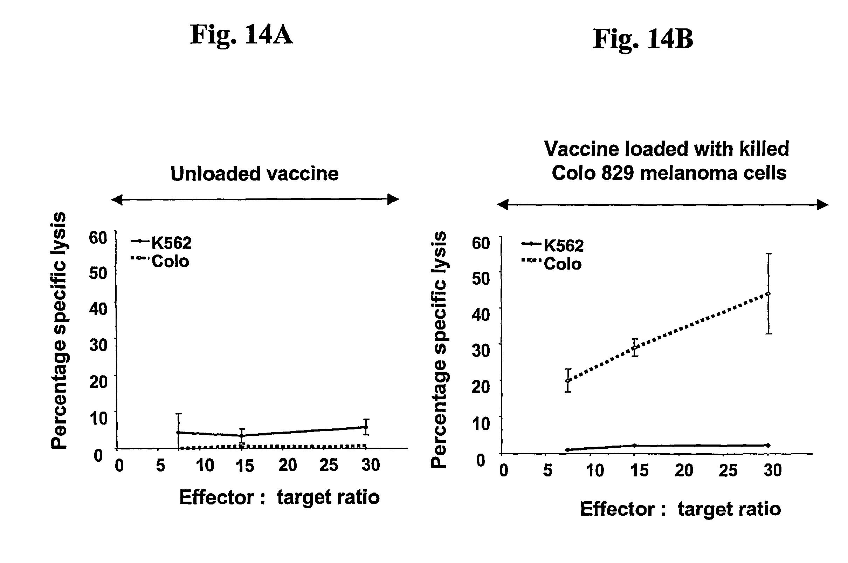

Figs. 14A and 14B depict the capacity of monocytes cultured for 3 days with (Fig. 14B) or without (Fig. 14A) killed tumor cells and with GM-CSF/IL-4 to induce differentiation of autologous CD8+ T cells into cytotoxic T cells (CTLs) able to kill melanoma cells after 2 stimulations.

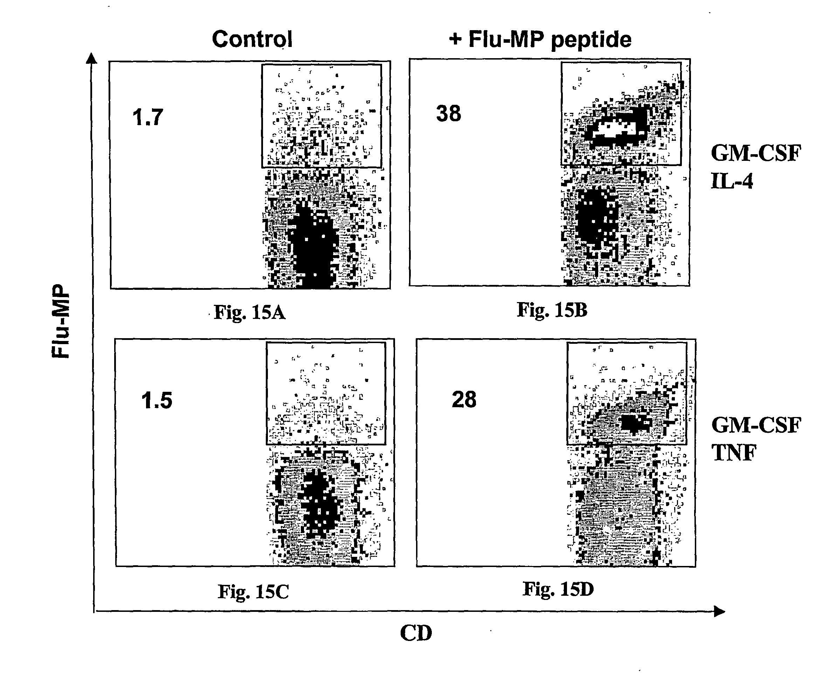

Figs. 15A-15D depict the capacity of GM-CSF/TNF alpha DCs to induce peptide-specific recall CD8 T cell responses. Flu-MP peptide pulsed GM-CSF/IL-4 DCs or GM-CSF/TNF alpha DCs were cultured at 1:10 ratio with autologous CD8 T cells and cytokine supplement (IL-7 and IL-2, 10U/ml). At Day 10, the T cells were labeled with anti-CD8 (abscissa) and Flu-MP tetramer (ordinate) to determine the expansion of specific CD8 T cells. Flu-MP peptide pulsed GM-CSF/TNF alpha DCs are shown to be efficient in inducing expansion of Flu-MP specific CD8 T cells in the 10-day culture (Fig. 15D).

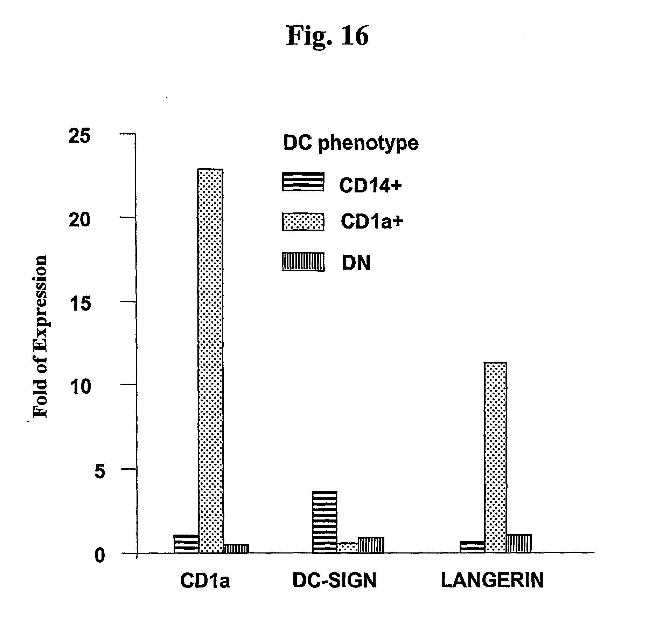

Fig. 16 depicts the DC gene expression patterns in terms of surface phenotypes of distinct subsets of GM-CSF/TNF alpha DCs, showing fold-expression (ordinate) of indicated genes (abscissa) for CD14+ intDCs (horizontal stripe), CD1a+ LCs (dotted) and CD14-CD1a- DN-DCs (vertical stripes). The presented genes CD1a, DC-SIGN (also known as CD209), and Langerin (also known as CD207) were selected from statistically significant genes expressed differentially between these DC subsets. The CD14+ subset expressed DC-SIGN, indicative of intDC. The CD1a+ subset expressed Langerin, indicative of LC. The DN (double negative) subset did not significantly express any of the selected markers.

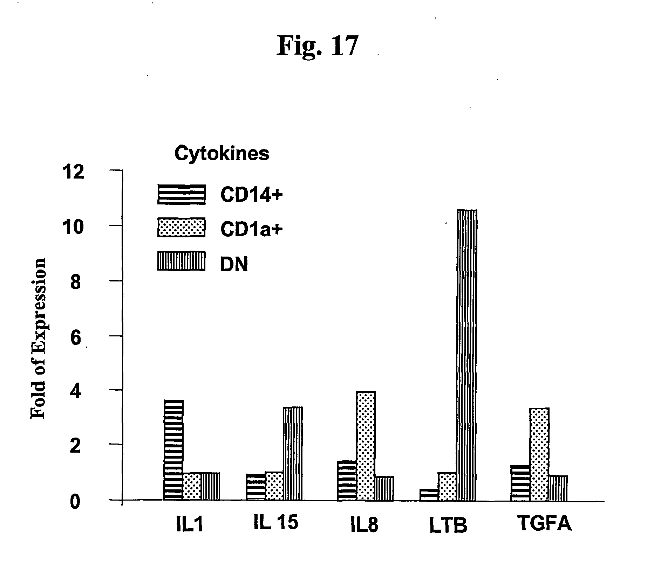

Fig. 17 depicts differential expression of cytokine mRNA of distinct subsets of GM-CSF/TNF alpha DCs, showing fold-expression (ordinate) of indicated genes (abscissa) for CD14+ intDCs (horizontal stripe), CD1a+ LCs (dotted) and CD14-CD1a-DN-DCs (vertical stripes). The DC subsets showed significant differential expression of the cytokines IL-1, IL-15, IL-8, LTB (lymphotoxin beta) and TGFA (transforming growth factor alpha), with the CD14+ subset expressing IL-1; CD1a+ DCs subset expressing predominantly TGFA and IL-8; and the DN subset expressing IL-15 and LTB.

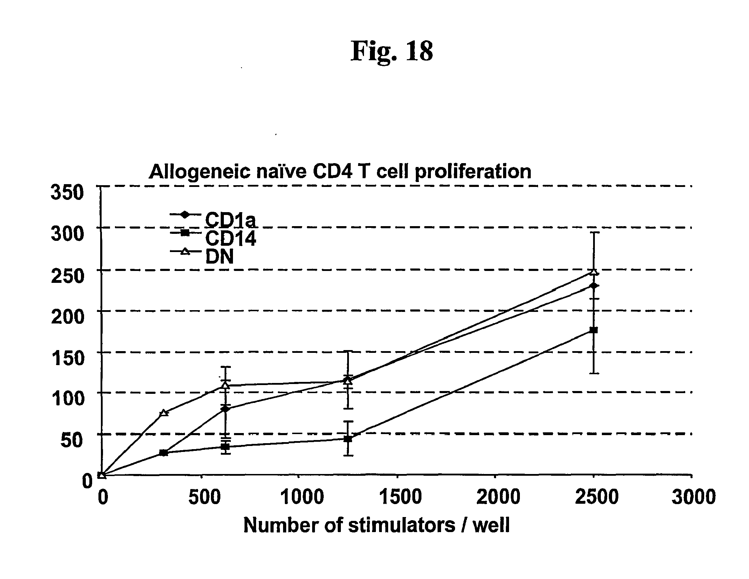

Fig. 18 depicts the capacity of GM-CSF/TNF alpha DC subsets to induce vigorous proliferation of naive allogeneic CD4 T cells. Total DC culture or sorted subsets are plated at graded doses (abscissa) with naïve (CD45RA+CCR7+CD45RO-) CD4+ T cells. Proliferation is measured by thymidine incorporation (ordinate) at Day 5. All subsets were capable of priming naive CD4 T cells; however, CD4 T cell proliferation in cultures with intDCs (CD14+) is less pronounced.

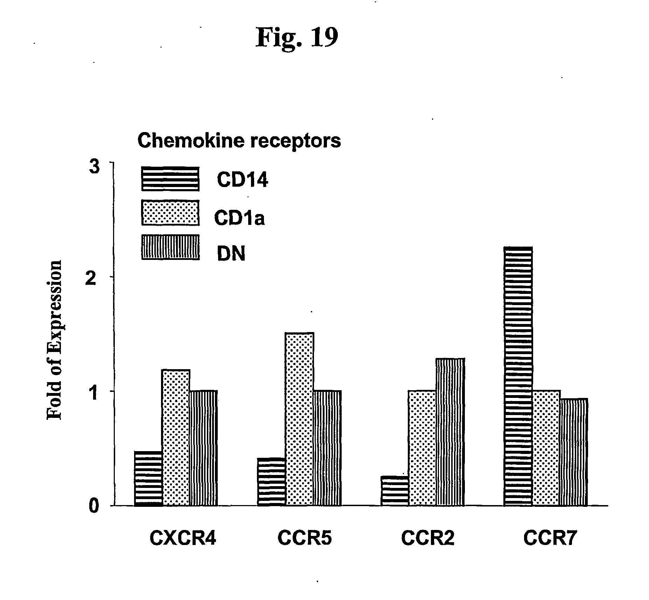

Fig. 19 depicts differential expression of chemokine receptors in CD4 T cells primed by distinct subsets of GM-CSF/TNF alpha DCs, showing fold expression (ordinate) in cultures with intDCs (CD14, horizontal stripes), CD1a DCs (LCs, dotted) or DN-DCs (vertical stripes).

### DETAILED DESCRIPTION

The present invention relates to compositions and methods for producing human antigen-presenting cells ex vivo. This invention relates more particularly to methods and compositions for producing antigen-presenting cells using an activator including but not limited to TNF alpha preferably in combination with a growth factor including but not limited to Granulocyte-Macrophage Colony Stimulating Factor (GM-CSF). This invention is particularly suited for producing dendritic cells from blood precursors ex vivo.

The present invention is a method for producing antigen-presenting cells from blood precursors such as monocytes ex vivo or in vitro for therapeutic (vaccine), diagnostic (immunomonitoring), and research (identification of tumor antigens) purposes comprising contacting blood precursors with a composition comprising an activator including but not limited to tumor necrosis factor (TNF) alpha preferably in combination with at least one growth factor including but not limited to GM-CSF. As used herein, TNF alpha is defined as TNF alpha or any TNF or TNF-like protein which functions as an activator in the methods of this invention. The formulation of growth and/or activation factors relevant to the present invention includes but is not limited to recombinant molecules and/or viral vectors and/or peptoids and/or any molecules that can trigger precursors to endogenous release of growth factors and/or activators. These dendritic cells, which include a mixture of interstitial DCs and Langerhans cells (LCs), can be sensitized to an antigen and administered to a patient for modulating an immune response to the antigen in the patient. The invention also includes a composition comprising dendritic cells produced according to this method. The antigenic material useful in the present invention includes soluble and particulate antigenic materials. When whole tumor cells are used for antigenic material, the cells may be either dead or dying, and either term is considered equivalent as used herein.

In one aspect, the invention is a one-step method for producing a vaccine from monocytes ex vivo comprising contacting monocytes with a composition comprising, for example, TNF alpha preferably in combination with at least one growth factor, for example, GM-CSF and at least one soluble or particulate antigen. The invention also includes a one-step vaccine produced according to this method. According to the methods of the present invention, antigen-loaded dendritic cell vaccines can be generated within as little as three (3) days. These dendritic cells are made by culturing monocytes and a soluble or particulate antigen(s) (for example, killed tumor cells) with a growth factor (for example, GM-CSF) and an activator (for example, TNF alpha). Importantly, reported generation of monocyte-derived dendritic cells as a cancer vaccine requires the use of GM-CSF or IL-3 combined with Interleukin 4 (IL-4) or IL-13. Both of these cytokines are strongly associated with polarization of T cell responses towards type 2 or regulatory T cells, which may not be desirable in the context of tumor vaccination. This invention, therefore, facilitates generation of dendritic cell vaccines, and such vaccines can be now used in large scale clinical trials.

It has now been found that pro-inflammatory cytokines such as GM-CSF and TNF alpha will promote monocyte differentiation into dendritic cells comprising at least four subsets of dendritic cells, i.e., Langerhans cells (LCs) having surface markers (CD1a+ CD 14- CD207+), interstitial DCs having surface markers (intDCs) (CD1a+ CD14+ CD207-), double negative DCs (DN-DCs) having surface markers (CD1a- CD14-), and a fourth subset having surface markers (CD1a- CD14+ CD209+). These dendritic cell precursors can capture antigen and subsequently differentiate ex vivo into antigen-loaded and antigen-presenting dendritic cell, which can be used in clinical trials. Further, it has now been found that that the three factors required to generate Dendritic cell vaccine, i.e. dendritic cell precursors, antigen(s) and cytokines can be put together simultaneously to rapidly generate antigen loaded dendritic cell vaccine. Unlike present technology which is time consuming and requires three steps (i.e., dendritic cell generation ex vivo from progenitors or precursors that takes 9 and 7 days, respectively; subsequent loading with antigens, and dendritic cell activation when the cells are generated from monocytes), the present invention allows rapid, all-in-one step generation of dendritic cell vaccine where all factors necessary in this process are combined.

According to the method of present invention, the vaccine is made from blood leukapheresis wherein monocytes are isolated either by adherence or by depletion or by positive selection. Such isolated monocytes are then cultured either in tissue culture bags or in plastic culture dishes or in Nunc Cell factories®. Cytokines are added at Day 1; antigens, for example, in the form of killed tumor cells are added either at Day 1 or at Day 2; and at Day 3, the vaccine is administered.

In another aspect of the present invention, the vaccine is frozen and used for boosting injection. In another aspect, the vaccine is used to make exosomes for boosting injections. In another aspect, exosomes made from this vaccine are used as the formulation of antigens to mix with monocytes and cytokines to make a vaccine.

According to the method of present invention, the vaccine is made from a blood unit wherein monocytes are isolated either by adherence or by depletion or by positive selection. Such isolated monocytes are then cultured either in tissue culture bags or in plastic culture dishes or in Nunc Cell factories®. Cytokines are added at Day 1; antigens, for example, in the form of killed tumor cells are added either at Day 1 or at Day 2; and at Day 3, the vaccine is administered. In this embodiment of the present invention, the vaccine is prepared freshly for each injection and the blood draw is made every month.

In one aspect, the invention is a method for producing antigen-specific CD4+ T lymphocytes in vitro or ex vivo comprising providing antigen-sensitized dendritic cells prepared by contacting blood precursors with a composition comprising, for example, TNF alpha preferably in combination with at least one growth factor, for example, GM-CSF to form dendritic cells, sensitizing the dendritic cells to at least one soluble or particulate antigen to form antigen-sensitized dendritic cells, and contacting the antigen-sensitized dendritic cells with a population of cells comprising T lymphocytes to form activated antigen-specific CD4+ T lymphocytes. These antigen-specific CD4+ T lymphocytes can be administered to a patient for modulating an immune response in the patient. The invention also includes a composition comprising antigen-specific CD4+ T lymphocytes produced according to this method.

In one aspect, the invention is a method for producing antigen-specific CD4+ T lymphocytes in vitro or ex vivo comprising providing antigen-sensitized dendritic cells prepared by contacting blood precursors with a composition comprising, for example, TNF alpha preferably in combination with at least one growth factor, for example, GM-CSF and at least one soluble or particulate antigen to form antigen sensitized dendritic cells and contacting the antigen-sensitized dendritic cells with a population of cells comprising T lymphocytes.to form activated antigen-specific CD4+ T lymphocytes. These antigen-specific CD4+ T lymphocytes can be administered to a patient for modulating an immune response in the patient. The invention also includes a composition comprising antigen-specific CD4+ T lymphocytes produced according to this method.

In one aspect, the invention is a method for producing antigen-specific CD8+ T lymphocytes in vitro or ex vivo comprising providing antigen-sensitized dendritic cells prepared by contacting blood precursors with a composition comprising, for example, TNF alpha preferably in combination with at least one growth factor, for example, GM-CSF to form dendritic cells, sensitizing the dendritic cells to an antigen to form antigen-sensitized dendritic cells, and contacting the antigen-sensitized dendritic cells with a population of cells comprising T lymphocytes to form activated antigen-specific CD8+ T lymphocytes. These antigen-specific CD8+ T lymphocytes can be administered to a patient for modulating an immune response in the patient. The invention also includes a composition comprising antigen-specific CD8+ T lymphocytes produced according to this method.

In one aspect, the invention is a method for producing antigen-specific CD8+ T lymphocytes in vitro or ex vivo comprising providing antigen-sensitized dendritic cells prepared by contacting blood precursors with a composition comprising, for example, TNF alpha preferably in combination with at least one growth factor, for example, GM-CSF and at least one soluble or particulate antigen to form antigen-sensitized dendritic cells and contacting the antigen-sensitized dendritic cells with a population of cells comprising T lymphocytes to form activated antigen-specific CD8+ T lymphocytes. These antigen-specific CD8+ T lymphocytes can be administered to a patient for modulating an immune response in the patient. The invention also includes a composition comprising antigen-specific CD8+ T lymphocytes produced according to this method.

### General Methods:

The methods for rapid generation of antigen-loaded DCs of the present invention used herein are detailed below. It is understood that these methods are meant to be representative and that modification such as known in the art are contemplated as part of the present invention.

Cell cultures. Mononuclear cells are isolated from peripheral blood of healthy volunteers or patients with advanced melanoma by Ficoll-Paque density gradient centrifugation. Monocytes are enriched either by depletion using microbeads or by a 2-hour plastic adherence method. Monocytes are seeded at 1x10⁶ cells/well in 6-well plates in 3ml of RPMI 1640 medium supplemented with 2mM L-Glutamine, 200 UI/ml penicillin, 200µg/ml streptomycin and a serum supplement of either 10% FBS or 5% autologous serum. Killed tumor cells are added to the 6-well plates at a density of 1x10⁶ dead cells/well. The monocytes are cultured with GM-CSF (100ng/ml, Immunex, Seattle, WA) and TNF alpha (20ng/ml, R&D) for 3 days.

### Killing of tumor cell lines.

Killed tumor cells can be prepared from a variety of cancer cell lines. The sources of tumor cells include but are not limited to those listed herein, and the selection of additional cell lines and the variations in methods to kill these cells are well within the knowledge of those skilled in the art. Methods to kill cancer cells useful in the present invention include but are not limited to conditions causing DNA damage such as irradiation, or receptor-mediated cell death via either Fas-ligation, TNF, or TRAIL. Exemplary methods are given below.

Once at confluence, the cell lines are harvested and cultured in small flask at a density of 1x10⁶ cells/ml. For the 1806 breast cancer cell line as well as the Colo 829 melanoma cell line, after a sensitization step with TNF-alpha (used at 100ng/ml), the cells were irradiated for 90 min and cultured for 48 hours in a serum free medium. For the Me275 melanoma cell line, the cells were cultured for 48 hours with betulinic acid (BA) (10µg/ml, Sigma Chemical Co., St. Louis, MO). The cell death was evaluated by a double staining FITC-Annexin V and Propidium iodide, and by Trypan blue exclusion (<25% viability).

Flow Cytometry Analysis (FACS) analysis. After 3 days of culture, the cells were processed for double staining (30 min at 4°C) using FITC-CD1a and PE-CD14, PE-CD80, PE-CD83 or PE-CD86.

Autologous proliferation assay. After 3 days of culture, the cells were used as stimulators for autologous CD4 T cells. Graded doses of stimulators cells were seeded with 1x10⁵ CD4+ T cells in round-bottomed microtest tissue-culture plates in complete RPMI 1640 with 10% human AB serum. After 4 days incubation, cells were pulsed overnight with 1µCi of [3H] thymidine, to determine T cell proliferation.

Cytotoxic T Lymphocyte (CTL) generation assay. After 3 days of culture, the cells were used as stimulators for autologous CD8 T cells and/or autologous peripheral blood lymphocytes (PBL). Cultures were restimulated every week and supplemented in the first week with IL-7 and in the following weeks with IL-2. CTL activity was tested in a standard chromium release assay using melanoma cells from which tumor bodies were generated as targets.

### Generation of Vaccine

The vaccine of the present invention useful for generating immune response-capable DCs is a significant advance over reported methods which require multiple steps and a long culture time. The following describes a detailed exemplary procedure for generating the one-step vaccine. It should be understood modifications or variations in culture conditions and preparation methods within the ability of those skilled in the art are contemplated as part of the invention.

Mononuclear cells are isolated from peripheral blood of healthy volunteers or patients with advanced melanoma. Prior to blood collection, recombinant human G-CSF (Amgen, Thousand Oaks, CA) can optionally be administered to the individual for peripheral blood stem cell mobilization (Banchereau et al., 2001). Monocytes are collected using Ficoll-Paque density gradient centrifugation, and suspended in CM medium. Monocytes can be enriched by adherence onto plastic dishes (Falcon 6-well, Franklin Lakes, NJ) for 2 hours at 37 degrees C. Alternatively, monocytes can be enriched by removing T cells, B cells, Natural Killer cells, and granulocytes using the Dynal® Monocyte Negative Isolation Kit (Dynal, Lake Success, NY) according to manufacturer's procedure.

Monocytes are seeded at 1x10⁶ cells/well in 6-well plates (Falcon) in 3ml of RPMI 1640 medium supplemented with 2mM L-Glutamine, 200 UI/ml penicillin, 200µg/ml streptomycin (all from Sigma Chemical Co., St. Louis, MO) and a serum supplement of either 10% fetal bovine serum (FBS, Hyclone) or 5% autologous serum (R&D). The monocytes were cultured with GM-CSF at 100ng/ml (Immunex, Seattle, WA) and TNF alpha at 20ng/ml (R&D) for 3 days. Killed tumor cells were added to the 6-well plates at a density of 1x10⁶ dead cells/well. In another embodiment, the killed tumor cells are added at Day 2 instead of Day 1 without affecting the immune response capability of the DCs. The culture is incubated for 3 days at 37 degrees C.

The culture can be evaluated by a number of methods: (1) examination of morphology of DCs; (2) the presence of cell markers by FACS analysis described below; (3) the ability to promote proliferation of CD4+/CD8+ T cells as described below in Autologous Proliferation Assay; and (4) the ability to induce cytotoxic T cell-induced lysis of tumor cells as described in Cytotoxic T Lymphocyte (CTL) Generation Assay.

### Example 1: Monocytes Cultured for 3 Days with GM-CSF and TNF alpha Give Rise to Two Subsets of Dendritic Cells Capable of Capturing Antigen and Inducing Immune Response

### Two types of DCs generated from monocytes cultured for 3 Days with GM-CSF and TNF alpha

Monocytes were isolated by depletion and cultured for three (3) days with either GM-CSF/IL-4, or with GM-CSF/TNF alpha. Both GM-CSF/IL-4 and GM-CSF/TNF alpha cultures displayed typical dendritic cell morphology characterized by large and delicate processes or veils in many directions protruded from cell bodies when viewed alive under phase-contrast microscopy (Banchereau and Steinman 1998). Cultured monocytes develop cellular marker at the stimulation of cytokines. These new cell markers can be observed by staining cells with fluorescent dye -labeled monoclonal antibodies, and viewed under fluorescent microscopy for the morphology and cellular distribution. Alternatively, percentage of cells in a population with a certain type of marker can be analyzed by flow cytometry as in Figs. 1A-1F.

As expected in control cultures with GM-CSF and IL-4 (hereinafter GM-CSF/IL4-DCs), the Day 3 cells became CD14- (negative), and approximately 50% of the cells acquired CD1a expression consistent with DC differentiation (Fig. 1E) which are destined to become intDCs. The Day 3 culture with GM-CSF and TNF alpha (hereinafter GM-CSF/TNF-DCs) were different, and two separate populations which exclusively express CD1a or CD14 were observed. The CD1a+CD14- and CD1a-CD14+ cells represents approximately 20% and 24% of the cell population, respectively, representing the Langerhan DC and the intDCs, respectively (Fig. 1B).

Another set of markers can be used to differentiate the Langerhans cells and interstitial DCs, with Langerhans cells (LCs) expressing only Langerin marker, while the interstitial DCs (intDCs) expressing only DC-SIGN. When stained with FITC labeled DC-SIGN or PE-CD207, a fluorescent dye, a cellular population could be examined the presence or absence of these two markers and their percentage can be calculated when assisted by flow cytometry. As shown in Fig. 2B, the Day 3 monocyte culture with GM-CSF/TNF alpha contained cells differentially expressing DC-SIGN (%) and Langerin (%), indicating the formation of both the Langerhans cells and the interstitial cells. In contrast, the Day 3 monocytes cultured in the presence of GM-CSF/IL4 were stained only by FITC-DC-SIGN but not by PE-CD207/Langerin, indicating that only Langerhan cells were generated (Fig. 2A). The above results showed that TNF induced monocyte differentiation into two subsets of dendritic cells: Langerhans cells and interstitial DCs.

### DCs induced by GM-CSF/TNF alpha are efficient in antigen capture

The present invention showed that the dendritic cells generated in the presence of GM-CSF/TNF alpha are as efficient in antigen capture as the current method using GM-CSF/IL4.

The primary function of immature DCs is to capture antigens as the initial first step in the process of capture of antigens and presenting antigens to cell surface. DCs capture antigens by several different methods including (1) phagocytosis capable of capturing large particulates or (2) receptor mediated endocytosis. Fig. 3A-3F illustrate the ability of dendritic cells generated in the presence of GM-CSF/TNF or GM-CSF/IL-4 to capture soluble antigens or large particulates. For soluble antigens, FITC-labeled Dextran (Molecular Probes, Inc., Eugene, OR) was used as described previously (Caux et al. 1997. "CD34+ hematopoietic progenitors from human cord blood differentiate along two independent dendritic cell pathways in response to granulocyte-macrophage colony-stimulating factor plus tumor necrosis factor alpha: II. Functional analysis," Blood 90:1458-1470). For large particulate antigens, dying tumor cells (melanoma or breast cancer) were harvested, washed with phosphate buffered saline (PBS), and labeled with the DNA specific fluorescent dye 7-aminoactinomycin (7-AAD) (Sigma Chemical Co., St Louis, MO) as previously described (Nouri-Shirazi, et al. 2000. "Dendritic cells capture tumor cell bodies and process/present their antigens to elicit primary immune responses," J Immunol 165 :3797-3803 ; and Berard, et al. 2000. "Priming of naïve CD8 T cells against melanoma antigens using dendritic cells loaded with killed allogeneic melanoma cells," J Exp Med 192:1535-1544). The immature DCs were harvested, washed and labeled with monoclonal antibody against CD11c which is labeled with fluorescent dye phycoerythrin (BDIS) using routine procedure. After staining, DC were washed and resuspended at a concentration of 2x10⁵ cells/ml. The FITC-Dextran was cocultured with the DC cells at a concentration of 1mg/ml at either 4 degrees C or 37 degrees C. While the killed tumor cells were cocultured with DC cells at 1:1 ratio for 1 hour at 37 degrees C.

The loading of FITC-dextran or the 7-AAD labeled tumor cell bodies by the DCs was measured by flow cytometry as the percentage of DC doubly positive for CD11c and dextran (Figs. 3A, 3B, 3D and 3E) or for CD11c and 7-AAD (Figs. 3C and 3F). Day 3 GM-CSF/TNF-DCs captured both soluble (FITC-Dextran) and particulate (killed tumor cells) antigens at similar levels in comparison to the GM-CSF/IL-4-DCs.

### Day 3 DCs generated by GM-CSF/TNF efficiently initiate immune reaction

The present invention showed that the Day 3 DCs generated by GM-CSF/TNF method efficiently initiate immune reaction by inducing proliferation of both CD4+ and CD8+ T cells.

After capturing and processing antigens, immature DCs matures and gain the ability to induce immune response. DCs can stimulate the outgrowth and activation for a variety of T cells, for instance stimulating the CD8 T cells into cytotoxic T cells via MHC class I bearing DCs, or to stimulate the CD4+ T cells into helper T cells via MHC Class II bearing DCs (Banchereau and Steinman 1998).

In the proliferation assay, the Day 3 DCs generated from GM-CSF/TNF or GM-CSF/IL4 were seeded in 96 well plates in graded doses from 0 to 2500 antigen-presenting cells (APC) per well. Purified CD4+ and CD8+ cells were obtained from Ficoll-separated PBMC of healthy individuals and depleted of other cell types by various monoclonal antibodies (Nouri-Shirazi, et al. 2000. "Dendritic cells capture killed tumor cells and present their antigens to elicit tumor-specific immune responses," J Immunol 165:3797-3803), and added to the DC-containing wells at 5x10⁴/well/200 µl. After four days of incubation, tritiated thymidine (Wallace, Inc., Gaithersburg, MD) was added at the activity of 1 µCi/well. The plates were harvested 16 hours later and the incorporated radioactivity was measured by liquid scintigraphy according to manufacturer's instructions.

As shown in Fig. 4, Day 3 GM-CSF/TNF-DCs were able to initiate an immune reaction as demonstrated by the induction of allogeneic CD4 T cell proliferation as efficiently as the GM-CSF/IL4 DCs, and this ability appears dose dependent. Both DCs from GM-CSF/TNF alpha and GM/CSF/IL4 were able to stimulate the proliferation of CD8 type T cells at a relatively low number of cells (Fig. 5).

In summary, the present invention demonstrated that the monocytes cultured for 3 days with GM-CSF and TNF alpha produced both Langerhan cells and intDCs, and these DCs are capable of capturing antigens and inducing immune response.

### Example 2: One-step Vaccine Gives Rise to Two Subsets of Dendritic Cells Capable of Capturing Antigen and Inducing Immune Response

The present invention presents a novel one-step method that capable of generating DC cells capable of immune response in only three days. In contrast to the reported method for making dendritic cells from monocytes which requires multiple steps and long term incubation, the method of the present invention comprises simultaneously incubating monocytes with antigenic material in the presence of GM-CSF/TNF alpha, and the antigens are presented to the DCs at an early stage. The DCs generated by this method are mature by several measures: (1) captured and processed antigens; (2) the capacity to induce the proliferation of CD4 and CD8 cells; and 3) the capacity to present antigens to T cells. The following describes different aspects of this one-step vaccine.

The ability of this one-step vaccine to capture antigen was demonstrated by mixing monocytes with killed tumor cells and culturing for 3 days in the presence of GM-CSF with or without adding IL4 or TNF alpha. At Day 3, Giemsa stained cells showed the capture of killed tumor cells. Particularly, in GM-CSF/TNF cultures at Day 3, very few uncaptured tumor bodies were left.

Day 3 GM-CSF/TNF cells cultured with killed tumor cells retained DC properties, and they were able to induce proliferation of allogeneic T cells both CD4 (Figs. 6A and 6B) and CD8 types (Figs. 7A and 7B). Day 3 GM-CSF/TNF cells cultured with killed tumor cells were more capable in stimulating CD8 type T cell proliferation than DCs generated in the absence of antigens. (Fig. 7B). More importantly, GM-CSF/TNF-DCs were able to present tumor cell antigens as demonstrated by their capacity to induce proliferation of autologous CD4 T cells (Fig. 8B). Those DCs that have seen antigen as generated via this one-step vaccine are far superior in presenting tumor cell antigens to the autologous CD4 T cells.

Detailed phenotypic analysis of monocytes cultured with tumor bodies and GM-CSF/TNF alpha showed that monocytes retain their capacity to differentiate into two subsets of dendritic cells, i.e, Langerhans cells and interstitial DCs (Fig. 9A-9L). Optimal differentiation was seen at the monocyte:killed tumor cell ratio of 1:0.1. At higher monocyte:tumor bodies ratios such as 1:1 and 1:0.3, the Langerhan cell proportion as demonstrated by the presence of Langerin marker diminishes. Killed tumor cells can be added to monocyte cultures also at Day 2 as shown in Figs. 10A-10I, where monocytes differentiate into Langerhans cells and interstitial DCs. Forward scatter/side scatter flow cytometry plots indicate that the cell population contain both CD14+ types and CD1a types indicating both Langerhan and intDCs (Fig. 10E-10F). These experiments illustrate that adding antigen at either Day 1 or Day2 does not affect the process of generating the two types of dendritic cells. In addition, these experiments illustrate the need to experimentally determine the ratio of a particular antigen using current dilution titer as a guide.

Cells of the one-step vaccine were also able to present tumor cell antigens to a mix of immune effectors. Indeed, as shown in Fig. 11, monocytes cultured with killed tumor cells and GM-CSF/TNF alpha for 3 days were able to induce proliferation of autologous peripheral blood lymphocytes. In this case, the monocyte:tumor bodies ratio is optimum at 1:0.3.

### One-step Vaccine induces Cytotoxic T cells to kill melanoma

The one-step vaccine of the present invention demonstrated the capacity to induce a cytotoxicity effect against melanoma. The generation of the one step vaccine is as described in Examples 1 and 2, with the exception that killed melanoma Colo 829 cells were present in the monocyte culture as the antigenic material.

Once at confluence, the Colo 829 melanoma cell lines were harvested and cultured in small flask at a density of 1x10⁶ cells/ml. After a sensitization step with TNF-alpha (100 ng/ml), the cells were irradiated for 90 min and cultured for 48 hours in a serum free medium. The cell death was evaluated by a double staining FITC-Annexin V and Propidium iodide, and by Trypan blue exclusion (<25% viability).

CTL generation assay. After 3 days of culture, the cells were used as stimulators for autologous CD8 T cells and/or autologous peripheral blood lymphocytes (PBL). Cultures were restimulated every week and supplemented in the first week with IL-7 and in the following weeks with IL-2. CTL activity was tested in a standard chromium release assay using melanoma cells from which tumor bodies were generated as targets.

Finally, cells of the one-step vaccine induced autologous CD8T cells to differentiate into CTLs able to kill melanoma cells that have been used as antigen (Figs. 12A, 12B, 13A, 13B, 14A and 14B). The vaccines which were not pulsed with antigen (killed Colo 829 melanoma cells), were incapable of inducing a cytotoxic T cell -mediated lysis of tumor cells of either the K562 and Colo melanoma tumors. The one-step vaccine which was pulsed with killed Colo 829 melanoma cells selectively caused the lysis of melanoma tumor cells by stimulation of the cytotoxic T cells but not the control tumor cell line K562. Stimulating the cytotoxic T-cells twice was as effective as three stimulations (Figs. 13B and 14B.). Furthermore, the ability of this one step vaccine to induce cytotoxic T cell mediated melanoma cells was similar to the GM-CSF/IL-4 vaccine of previously reported method.

Using the method shown here, other types of tumor cells can be used to pulse the DC cells, and the toxicity on these tumors could be measured using the assay outlined here, and the optimum ratio for effector to target ratio could be determined experimentally using the methods and range outlined herein. Overall, the data presented herein demonstrated the ability of one-step vaccine against melanoma in treating or eradicating the melanoma. Furthermore, it demonstrates the potential of this method in treating other types of cancer using methods outlined herein.

### Example 3: Further Characterization of Dendritic Cells Derived from Monocytes Cultured for 3 Days with GM-CSF and TNF alpha

In another study, monocytes were isolated by depletion and cultured for 3 days with GM-CSF/TNF alpha. The resulting GM-CSF/TNF-DCs were able to present antigens to autologous T cells. As shown in Fig. 15D, Flu-MP peptide pulsed GM-CSF/TNF-DCs were efficient in inducing expansion of Flu-MP specific CD8 T cells in one-week culture.

Upon further examination, the GM-CSF/TNF-DCs were found to consist of distinct subsets with different biological properties: LCs (CD1a+ CD14- CD207+), intDCs (CD1a+ CD14+ CD207-) and DN-DCs (CD1a- CD14-). The subsets are separated after surface staining with antibodies against indicated surface markers CD1a (Biosource), CD207 (Beckman-Coulter), CD14 (BDIS) and sorting using flow cytometry (FACSVantage, BDIS). Microarray analysis confirmed that these DC subsets display unique molecular signatures that may translate into unique biological functions. The preliminary analysis of specific gene families indicated differential expression of antigen processing molecules, chemokines and their receptors, cytokines and their receptors and adhesion molecules. A few examples of differential expression are given in Figs. 16 and 17. As shown in Fig. 16, consistent with surface phenotype the CD1a+ subsets of GM-CSF/TNF-DCs expressed high levels of CD1a and Langerin mRNA (CD207+) indicating Langerhans cell differentiation. The CD14+ subset expressed DC-SIGN mRNA (CD209+) consistent with interstitial DC differentiation. The DN-DCs did not significantly express any of the selected markers. As shown in Fig. 17, the three subsets expressed different cytokines with CD1a+ DCs expressing predominantly TGF-alpha and IL-8, while CD 14+ DCs expressed IL-1. Interestingly, DN-DCs expressed high levels of lymphotoxin beta (LTB) mRNA and IL-15, a cytokine essential for maturation of CD8+ effector/memory T cells.

Distinct subsets of GM-CSF/TNF-DCs induced different T cell responses, thus indicating different functions. Sorted subsets of non-activated GM-CSF/TNF-DCs were cultured with naïve allogeneic CD8 or CD4 T cells. T cell responses were analyzed at two levels: (a) proliferation after 5 days of culture (tritiated thymidine incorporation), and (b) global mRNA analysis using Affymetrix microarray chips. All subsets of GM-CSF/TNF-DCs induced vigorous proliferation of naive allogeneic CD8 T cells. All subsets also were capable of priming naive CD4 T cells; however CD4 T cell proliferation in cultures with intDCs (CD14+) was less pronounced (Fig. 18). The analysis of global mRNA expression in CD4 T cells showed striking differences in CD4 T cell response to intDCs (CD14+) as compared to other GM-CSF/TNF-DC subsets. For example, we observed differential expression of chemokine receptors (CXCR4, CCR5, CCR2 and CCR7) in CD4 T cells primed by distinct subsets of GM-CSF/TNF-DCs (Fig. 19) indicating that these T cells may display different migratory capacity.

Upon further investigation of the GM-CSF/TNF-DCs, a fourth subset was characterized as (CD1a- CD14+ CD209+).

### Example 4: Treating Patients with Melanoma with Monocyte-derived Vaccine

To demonstrate treatment of patients with melanoma with the monocyte derived vaccine of the present invention, patients with Stage IV melanoma as proven by biopsy are administered the vaccine in a similar protocol to the a trial for CD34+ progenitor derived DCs (Banchereau et al. 2001. "Immune and clinical responses in patients with metastatic melanoma to CD34(+) progenitor-derived dendritic cell vaccine," Cancer Res 61:6451-6458).

To prepare the vaccine, monocytes are isolated from peripheral blood of each patient by Ficoll-Paque density gradient centrifugation, enriched by depletion or adherence method, and seeded onto microplates with a culture medium containing GM-CSF and TNF alpha. The vaccine is made by one of the following methods: (1) on Day 1, melanoma cells (from cell line Colo829 or from each individual patient) are killed by a preferred method known in the art and added to the culture medium with monocytes and GM-CSF/TNF alpha; (2) on Day 1, all cells are exposed to Keyhole limpet hemocyanin (KLH), and a subset of cells, i.e. 20% , are pulsed with restricted influenza matrix peptide (Flu-MP), and the remaining cells (i.e. 80%) pulsed with peptides derived from four melanoma antigens such as MalanA/MART-1, tyrosinase, MAGE-3 and gp100; (3) on Day 2, melanoma cells (from cell line Colo829 or from each individual patients) are killed by irradiation and added to the culture medium with monocytes and GM-CSF/TNF alpha; or (4) on Day 2, all cells are exposed to Keyhole limpet hemocyanin (KLH), and a subset of cells, i.e. 20% , are pulsed with restricted influenza matrix peptide (Flu-MP), and the remaining cells (i.e. 80%) pulsed with peptides derived from four melanoma antigens such as MalanA/MART-1, tyrosinase, MAGE-3 and gp 100. At Day 3 of the culture, the antigen loaded antigen presenting cells are harvested, washed, and formulated into a vaccine. The vaccine is then evaluated by criteria including but not limited to the following using procedures well defined in Example 1 and 2: DC morphology; a minimum of at least 20% DCs in cell preparation as determined by phenotypic analysis (the remaining portion contains DC precursors, etc.); and/or reactivity with a panel of monoclonal antibodies to determine cell markers, and /or the ability to stimulate lymphocyte reactions prior to use in the patient.

The patient is treated with the vaccine of the present invention in a 6 week long out-patient course and vaccinated every 14 days for a total of four vaccinations. The vaccine is administered to the patient at three injection sites including thighs and upper arms. The immunological monitoring is carried out at least two time points before vaccination, as well as at 5 and /or 14 days after each vaccination and at 14 or 28 days after the fourth vaccination. All patients undergo assessment of tumor status prior to trial and four weeks after the fourth vaccination. Disease progression is defined as more than 25% increase in target lesions and/or the appearance of new lesions. The analysis of patients response is divided into several groups, response to at least one melanoma antigen; response to multiple melanoma antigens, response to the killed whole melanoma cells; and response to the control antigens such as KLH and Flu-MP. The immunological response is correlated with the tumor progression data.

### Example 5: Induction of antigen-specific non-responsiveness with monocyte-derived antigen loaded antigen-presenting cells

Dendritic cells made according to the present invention are loaded with antigenic material to which T cells can be rendered non-responsive. In this method, T cells are exposed to a CD14+ CD1a- subset of GM-CFS and TNF alpha generated antigen loaded DCs. In this way, the T cells can recognize the antigen but will become non-responsive and are unable to proliferate when restimulated with the same antigen.

Purified CD4+ T cells are obtained from Ficoll-separated PBMC of healthy volunteers and depleted of other cells using CD8 (B9.11), CD14 (RMO52), CD16 (3G8), CD19 (J4.119), CD56 (NKH-1), anti-HLA-DR (B8.12.2), anti-glycophorin A (D2.10) monoclonal antibodies (mAbs) (Beckman-Coulter, Miami, FL) and Goat anti-mouse IgG Dynabeads (Dynal, Lake Success, NY). The purity of the enriched populations was > 90%. Antigen-loaded DCs are prepared and used to stimulate naïve CD4 T cells as described herein.

A tolerance assay can be performed wherein purified CD4+ T cells (10⁶/well) are cocultured in 24-well plates with a CD14+ CD1a- subset of monocyte-derived DCs prepared with TNF alpha and GM-CSF as described herein. After 5 days, the T cells are harvested, washed and rested for an additional 2 days. T cells from primary cultures are rechallenged in a proliferation assay with appropriate antigens, wherein proliferation is determined by uptake of (1µCi/well for the last 16 hr) tritiated thymidine.

In summary, the present invention is a method of making a dendritic cell vaccine in three days. In a preferred method, dendritic cells are generated and loaded with antigen in one step, with the antigen-loaded dendritic cell vaccine available in three days. In another method, the generation of dendritic cells and antigen-loading are performed in two steps, with the antigen-loaded dendritic cell vaccine available in three days. In a preferred method, the dendritic cell vaccine is prepared from blood leukapheresis. In another preferred method, the vaccine is prepared from a blood unit. In a preferred method, the vaccine can be made by exposing precursors to chemicals/peptides/peptoids mimicking cytokine(s). In another preferred method, the vaccine can be made using molecules and/or particles triggering cytokine release from monocytes. A dendritic cell vaccine made according to a preferred method of the present invention is derived from monocytes using an activator including but not limited to TNF alpha preferably in combination with a growth factor including but not limited to Granulocyte-Macrophage Colony Stimulating Factor (GM-CSF), and without the use of either interleukin 4 or interleukin 13. Preferably, the dendritic cell vaccine made according to a preferred method of the present invention contains Langerhans cells and is derived from monocytes using an activator including but not limited to TNF alpha preferably in combination with a growth factor including but not limited to Granulocyte-Macrophage Colony Stimulating Factor (GM-CSF), and without the use of either interleukin 4, transforming growth factor beta, or interleukin 15. Regarding antigen-loading, the dendritic cell vaccines of the present invention are preferably loaded with soluble or particulate antigenic material including but not limited to soluble protein such as immune complexes or killed tumor cells. In another method, the dendritic cell vaccines of the present invention are loaded using exosomes.

According to these methods, the resulting vaccines are preferably composed of two subsets of dendritic cells from blood precursors, including but not limited to Langerhans cells and interstitial dendritic cells. Dendritic cell vaccines made according to the methods of the present invention can be used for a variety of purposes including but not limited to the following: (1) treatment of diseases including but not limited to cancer, infectious diseases, viral diseases, and autoimmune diseases; (2) generating exosomes; (3) monitoring tumor specific immune responses in an in vitro assay; and (4) monitoring antigen-specific immune responses in an in vitro assay.

The present invention is also method for inducing cytotoxic immune responses against tumor cells using a one-step, three-day dendritic cell vaccine in vitro or in vivo. According to the methods of the present invention, cytotoxic immune responses can be induced against shared tumor antigens or against multiple tumor antigens. The resulting immune effector cells can be CD4 positive cells, CD8 positive cells, natural killer cells or natural killer T cells. In a preferred method, the immune effector cells are generated in vivo. In another preferred method, the immune effector cells are generated ex vivo and administered to patient. In another preferred method, the immune effector cells are generated for in vitro research purposes.

The following embodiments are disclosed in the application:
1. A method for producing antigen loaded antigen-presenting cells from monocytes ex vivo comprising simultaneously contacting said monocytes with soluble or particulate antigenic material, tumor necrosis factor alpha, and granulocyte-macrophage colony stimulating factor.
2. The method of embodiment 1, wherein said antigen loaded antigen-presenting cells are produced in less than four days.
3. A method for producing antigen loaded antigen-presenting cells from monocytes ex vivo comprising contacting said monocytes with tumor necrosis factor alpha and granulocyte-macrophage colony stimulating factor at one time point to form antigen-presenting cells and contacting said antigen-presenting cells with soluble or particulate antigenic material at a second time point to form antigen loaded antigen-presenting cells, wherein said antigen loaded antigen-presenting cells are produced in less than four days.
4. The method of embodiment 1, wherein said antigenic material consists of one or more materials selected from the group consisting of antigenic peptides, peptide mimetics, proteins, polyproteins, immune complexes, whole dying cell bodies, dying cell body fragments, viral vectors and liposomes.
5. The method of embodiment 2, wherein said antigenic material consists of one or more materials selected from the group consisting of antigenic peptides, peptide mimetics, proteins, polyproteins, immune complexes, whole dying cell bodies, dying cell body fragments, viral vectors and liposomes.
6. The method of embodiment 3, wherein said antigenic material consists of one or more materials selected from the group consisting of antigenic peptides, peptide mimetics, proteins, polyproteins, immune complexes, whole dying cell bodies, dying cell body fragments, viral vectors and liposomes.
7. The method of embodiment 1, wherein said antigenic material consists of soluble antigenic material.
8. The method of embodiment 2, wherein said antigenic material consists of soluble antigenic material.
9. The method of embodiment 3, wherein said antigenic material consists of soluble antigenic material.
10. The method of embodiment 1, wherein said antigenic material consists of whole dying cell bodies, dying cell body fragments or both.
11. The method of embodiment 2, wherein said antigenic material consists of whole dying cell bodies, dying cell body fragments or both.
12. The method of embodiment 3, wherein said antigenic material consists of whole dying cell bodies, dying cell body fragments or both.
13. The method of embodiment 1, 2, 3, 4, 5, 6, 7, 8, 9, 10, 11 or 12, further comprising separating said antigen loaded antigen-presenting cells into fractions containing one or more subsets selected from the group consisting of cells with surface markers (CD1a+ CD207+); cells with surface markers (CD1a+ CD207-); cells with surface markers (CD1a-CD14-); and cells with surface markers (CD14+ CD1a- CD209+).
14. A vaccine comprising antigen loaded antigen-presenting cells made according to the steps of simultaneously contacting monocytes ex vivo with soluble or particulate antigenic material, tumor necrosis factor alpha, and granulocyte-macrophage colony stimulating factor to form antigen loaded antigen-presenting cells.
15. The vaccine of embodiment 14, wherein said antigen loaded antigen-presenting cells are produced in less than four days.
16. A vaccine comprising antigen loaded antigen-presenting cells made according to the steps of contacting monocytes ex vivo with tumor necrosis factor alpha and granulocytemacrophage colony stimulating factor at one time point to form antigen-presenting cells and contacting said antigen-presenting cells with soluble or particulate antigenic material at a second time point to form antigen loaded antigen-presenting cells, wherein said antigen loaded antigen-presenting cells are produced in less than four days.
17. The vaccine of embodiment 14, 15 or 16, wherein said antigenic material consists of one or more materials selected from the group consisting of antigenic peptides, peptide mimetics, proteins, polyproteins, whole dying cell bodies and dying cell body fragments.
18. The vaccine of embodiment 14, 15 or 16, wherein said antigenic material consists of soluble antigenic material.
19. The vaccine of embodiment 14, 15 or 16, wherein said antigenic material consists of whole dying cell bodies, dying cell body fragments or both.
20. A vaccine comprising monocyte-derived antigen loaded antigen-presenting cells, wherein said antigen-presenting cells comprise Langerhans cells and interstitial dendritic cells.
21. A vaccine comprising monocyte-derived antigen loaded antigen-presenting cells, wherein said antigen-presenting cells consist of two or more subsets selected from the group consisting of cells with surface markers (CD1a+ CD207+); cells with surface markers (CD1a+ CD207-); cells with surface markers (CD1a- CD14-); and cells with surface markers (CD14+ CD1a- CD209+).
22. A method of inducing tumor specific immune response in a tumor-bearing patient comprising:
   producing a vaccine comprising antigen loaded antigen-presenting cells, said antigen loaded antigen-presenting cells made from monocytes ex vivo by simultaneously contacting said monocytes with soluble or particulate antigenic material possessing at least one tumor antigen, tumor necrosis factor alpha, and granulocyte-macrophage colony stimulating factor;
   administering said vaccine to said patient,
   wherein said patient's cells upon contact with said loaded antigen-presenting cells in said vaccine mature to form cytotoxic cells expressing cytotoxic activity against said tumor cells.
23. The method of embodiment 22, wherein said antigen loaded antigen-presenting cells are made in less than four days.
24. A method of inducing tumor specific immune response in a tumor-bearing patient comprising:
   producing a vaccine comprising antigen loaded antigen-presenting cells, said antigen loaded antigen-presenting cells made by contacting monocytes ex vivo with tumor necrosis factor alpha and granulocyte-macrophage colony stimulating factor at one time point to form antigen-presenting cells and contacting said antigen-presenting cells with soluble or particulate antigenic material possessing at least one tumor antigen at a second time point to form antigen loaded antigen-presenting cells, wherein said antigen loaded antigen-presenting cells are produced in less than four days;
   administering said vaccine to said patient,
   wherein said patient's cells upon contact with said loaded antigen-presenting cells in said vaccine mature to form cytotoxic cells expressing cytotoxic activity against said tumor cells.
25. A method of inducing tumor specific responses in a tumor-bearing patient comprising:
   isolating monocytes and cytotoxic cell precursors from said patient;
   producing antigen loaded antigen-presenting cells from said monocytes by simultaneously contacting said monocytes with soluble or particulate antigenic material possessing at least one tumor antigen, tumor necrosis factor alpha, and granulocytemacrophage colony stimulating factor;
   coculturing said loaded antigen-presenting cells with said isolated cytotoxic cell precursors under conditions for their maturation to form cytotoxic cells; and
   administering said cytotoxic cells to said patient,
   wherein said cytotoxic cells express cytotoxic activity against said tumor cells.
26. The method of embodiment 25, wherein said antigen loaded antigen-presenting cells are made in less than four days.
27. A method of inducing tumor specific responses in a tumor-bearing patient comprising:
   isolating monocytes and cytotoxic cell precursors from said patient;
   producing antigen loaded antigen-presenting cells by contacting monocytes ex vivo with tumor necrosis factor alpha and granulocyte-macrophage colony stimulating factor at one time point to form antigen-presenting cells and contacting said antigen-presenting cells with soluble or particulate antigenic material possessing at least one tumor antigen at a second time point to form antigen loaded antigen-presenting cells, wherein said antigen loaded antigen-presenting cells are produced in less than four days;
   coculturing said loaded antigen-presenting cells with said isolated cytotoxic cell precursors under conditions for their maturation to form cytotoxic cells; and
   administering said cytotoxic cells to said patient,
   wherein said cytotoxic cells express cytotoxic activity against said tumor cells.
28. A method of inducing an immune response in a patient comprising:
   producing a vaccine comprising antigen loaded antigen-presenting cells, said antigen loaded antigen-presenting cells made from monocytes ex vivo by simultaneously contacting said monocytes with soluble or particulate antigenic material possessing at least one antigen for which an immune response is desired, tumor necrosis factor alpha, and granulocyte-macrophage colony stimulating factor;
   administering said vaccine to said patient,
   wherein said patient's cells upon contact with said loaded antigen-presenting cells in said vaccine mature to form cells expressing immunologic activity against said antigen.
29. The method of embodiment 28, wherein said antigen loaded antigen-presenting cells are made in less than four days.
30. A method of inducing an immune response in a patient comprising:
   producing a vaccine comprising antigen loaded antigen-presenting cells, said antigen loaded antigen-presenting cells made by contacting monocytes ex vivo with tumor necrosis factor alpha and granulocyte-macrophage colony stimulating factor at one time point to form antigen-presenting cells and contacting said antigen-presenting cells with soluble or particulate antigenic material possessing at least one antigen for which an immune response is desired at a second time point to form antigen loaded antigen-presenting cells, wherein said antigen loaded antigen-presenting cells are produced in less than four days;
   administering said vaccine to said patient,
   wherein said patient's cells upon contact with said loaded antigen-presenting cells in said vaccine mature to form cells expressing immunologic activity against said antigen.
31. A method for mounting an immune response mediated by cytotoxic T cells comprising:
   producing antigen loaded antigen-presenting cells from monocytes ex vivo by simultaneously contacting said monocytes with soluble or particulate antigenic material possessing at least one antigen for which an immune response is desired, tumor necrosis factor alpha, and granulocyte-macrophage colony stimulating factor;
   coculturing said loaded antigen-presenting cells with naive T cells under conditions for T cell maturation to form cytotoxic T cells; and
   contacting said cytotoxic T cells with target cells possessing said antigen,
   wherein said cytotoxic T cells express cytotoxic activity against said target cells possessing said antigen.
32. The method of embodiment 31, wherein said antigen loaded antigen-presenting cells are made in less than four days.
33. A method for mounting an immune response mediated by cytotoxic T cells comprising:
   producing antigen loaded antigen-presenting cells by contacting monocytes ex vivo with tumor necrosis factor alpha and granulocyte-macrophage colony stimulating factor at one time point to form antigen-presenting cells and contacting said antigen-presenting cells with soluble or particulate antigenic material possessing at least one antigen for which an immune response is desired at a second time point to form antigen loaded antigen-presenting cells, wherein said antigen loaded antigen-presenting cells are produced in less than four days;
   coculturing said loaded antigen-presenting cells with naive T cells under conditions for T cell maturation to form cytotoxic T cells; and
   contacting said cytotoxic T cells with target cells possessing said antigen,
   wherein said cytotoxic T cells express cytotoxic activity against said target cells possessing said antigen.
34. The method of embodiment 22, 23, 24, 25, 26, 27, 28, 29, 30, 31, 32 or 33, wherein said antigenic material consists of soluble antigenic material.
35. The method of embodiment 22, 23, 24, 25, 26, 27, 28, 29, 30, 31, 32 or 33, wherein said antigenic material consists of whole dying cell bodies, dying cell body fragments or both.
36. The method of embodiment 22, 23, 24, 25, 26, 27, 28, 29, 30, 31, 32 or 33, wherein cytotoxic cells are CD4 positive.
37. The method of embodiment 34, wherein cytotoxic cells are CD4 positive.
38. The method of embodiment 35, wherein cytotoxic cells are CD4 positive.
39. The method of embodiment 22, 23, 24, 25, 26, 27, 28, 29, 30, 31, 32 or 33, wherein cytotoxic cells are CD8 positive.
40. The method of embodiment 34, wherein cytotoxic cells are CD8 positive.
41. The method of embodiment 35, wherein cytotoxic cells are CD8 positive.
42. A method for modifying an immune response in a patient comprising:
   producing antigen loaded antigen-presenting cells, said antigen loaded antigen-presenting cells derived from monocytes ex vivo by simultaneously contacting said monocytes with soluble or particulate antigenic material possessing at least one antigen for which an immune response is desired, tumor necrosis factor alpha, and granulocyte-macrophage colony stimulating factor;
   administering said antigen-presenting cells to said patient,
   wherein said patient's cells upon contact with said loaded antigen-presenting cells are modulated not to express immunologic activity against said antigen.
43. A method for producing an antigen loaded antigen presenting cell fraction composed of two or more subsets comprising:
   simultaneously contacting monocytes ex vivo with soluble or particulate antigenic material, tumor necrosis factor alpha, and granulocyte-macrophage colony stimulating factor to form antigen loaded antigen-presenting cells;
   isolating from said antigen loaded antigen-presenting cells two or more subsets wherein each subset is capable of eliciting a distinct T cell response and mounting a distinct immune response when administered to a patient;
   combining two or more said subsets to form said antigen loaded antigen presenting cell traction.

## Claims

1. A vaccine comprising antigen loaded antigen-presenting cells made according to the steps of simultaneously contacting monocytes *ex vivo* with soluble or particulate antigenic material, tumor necrosis factor alpha, and granulocyte-macrophage colony stimulating factor to form antigen loaded antigen-presenting cells, wherein said antigen-presenting cells comprise one or more subsets selected from the group consisting of Langerhans cells with surface markers (CD1a+ CD 14- CD207+); interstitial dendritic cells with surface markers (CD1a+ CD14+ CD207-); double-negative dendritic cells (DN-DCs) with surface markers (CD1a- CD14-); and dendritic cells with surface markers (CD1a- CD14+ CD209+), wherein said antigen loaded antigen-presenting cells are produced in less than four days.

2. A vaccine comprising antigen loaded antigen-presenting cells made according to the steps of contacting monocytes *ex vivo* with tumor necrosis factor alpha and granulocyte-macrophage colony stimulating factor at one time point to form antigen-presenting cells and contacting said antigen-presenting cells with soluble or particulate antigenic material at a second time point to form antigen loaded antigen-presenting cells, wherein said antigen loaded antigen-presenting cells are produced in less than four days, and wherein said antigen-presenting cells comprise one or more subsets selected from the group consisting of Langerhans cells with surface markers (CD1a+ CD 14- CD207+); interstitial dendritic cells with surface markers (CD1a+ CD14+ CD207-); double-negative dendritic cells (DN-DCs) with surface markers (CD1a-CD14-); and dendritic cells with surface markers (CD1a- CD14+ CD209+).

3. The vaccine of claims 1 or 2, wherein said antigenic material consists of one or more materials selected from the group consisting of antigenic peptides, peptide mimetics, proteins, polyproteins, soluble antigenic material whole dying cell bodies, or dying cell body fragments.

4. A vaccine comprising monocyte-derived antigen loaded antigen-presenting cells, wherein said antigen-presenting cells consist of two or more subsets selected from the group consisting of cells with surface markers (CD1a+ CD207+); cells with surface markers (CD1a+ CD207-); cells with surface markers (CD1a- CD14-); and cells with surface markers (CD14+ CD1a- CD209+).

5. Use of antigen-loaded antigen-presenting cells made from monocytes *ex vivo* by simultaneously contacting said monocytes with soluble or particulate antigenic material possessing at least one tumor antigen, tumor necrosis factor alpha, and granulocyte-macrophage colony stimulating factor, wherein said antigen-presenting cells comprise one or more subsets selected from the group consisting of Langerhans cells with surface markers (CD 1 a+ CD 14- CD207+); interstitial dendritic cells with surface markers (CD1a+ CD14+ CD207-); double-negative dendritic cells (DN-DCs) with surface markers (CD1a- CD14-); and dendritic cells with surface markers (CD1a-CD 14+ CD209+), to manufacture a vaccine for treating cancer in a patient,
wherein said patient's cells upon contact with said loaded antigen-presenting cells in said vaccine mature to form cytotoxic cells expressing cytotoxic activity against said tumor cells, wherein said antigen loaded antigen-presenting cells are made in less than four days.

6. Use of antigen loaded antigen-presenting cells made by contacting monocytes *ex vivo* with tumor necrosis factor alpha and granulocyte-macrophage colony stimulating factor at one time point to form antigen-presenting cells and contacting said antigen-presenting cells with soluble or particulate antigenic material possessing at least one tumor antigen at a second time point to form antigen loaded antigen-presenting cells, wherein said antigen loaded antigen-presenting cells are produced in less than four days, and wherein said antigen-presenting cells comprise one or more subsets selected from the group consisting of Langerhans cells with surface markers (CD1a+ CD 14-CD207+); interstitial dendritic cells with surface markers (CD1a+ CD14+ CD207-); double-negative dendritic cells (DN-DCs) with surface markers (CD1a- CD14-); and dendritic cells with surface markers (CD1a- CD14+ CD209+), to manufacture a vaccine for treating cancer in a patient,
wherein said patient's cells upon contact with said loaded antigen-presenting cells in said vaccine mature to form cytotoxic cells expressing cytotoxic activity against said tumor cells.

7. Use of cytotoxic cells to manufacture a medicament for inducing tumor specific immune response in a tumor-bearing patient, wherein said cytotoxic cells are produced by a method comprising:
producing antigen loaded antigen-presenting cells from monocytes isolated from said patient by simultaneously contacting said monocytes with soluble or particulate antigenic material possessing at least one tumor antigen, tumor necrosis factor alpha, and granulocyte-macrophage colony stimulating factor, wherein said antigen-presenting cells comprise one or more subsets selected from the group consisting of Langerhans cells with surface markers (CD1a+ CD 14- CD207+); interstitial dendritic cells with surface markers (CD1a+ CD14+ CD207-); double-negative dendritic cells (DN-DCs) with surface markers (CD1a- CD14-); and dendritic cells with surface markers (CD1a- CD14+ CD209+);
coculturing said loaded antigen-presenting cells with cytotoxic cell precursors isolated from said patient under conditions for their maturation to form cytotoxic cells,
wherein said cytotoxic cells express cytotoxic activity against said tumor cells and wherein said antigen loaded antigen-presenting cells are made in less than four days.

8. Use of cytotoxic cells to manufacture a medicament for inducing tumor specific immune response in a tumor-bearing patient, wherein said cytotoxic cells are produced by a method comprising:
producing antigen loaded antigen-presenting cells by contacting monocytes isolated from said patient with tumor necrosis factor alpha and granulocyte-macrophage colony stimulating factor at one time point to form antigen-presenting cells and contacting said antigen-presenting cells with soluble or particulate antigenic material possessing at least one tumor antigen at a second time point to form antigen loaded antigen-presenting cells, wherein said antigen loaded antigen-presenting cells are produced in less than four days, and wherein said antigen-presenting cells comprise one or more subsets selected from the group consisting of Langerhans cells with surface markers (CD1a+ CD14- CD207+); interstitial dendritic cells with surface markers (CD1a+ CD14+ CD207-); double-negative dendritic cells (DN-DCs) with surface markers (CD1a- CD14-); and dendritic cells with surface markers (CD1a-CD 14+ CD209+);
coculturing said loaded antigen-presenting cells with cytotoxic cell precursors isolated from said patient under conditions for their maturation to form cytotoxic cells,
wherein said cytotoxic cells express cytotoxic activity against said tumor cells.

9. Use of antigen loaded antigen-presenting cells made from monocytes *ex vivo* by simultaneously contacting said monocytes with soluble or particulate antigenic material possessing at least one antigen for which an immune response is desired, tumor necrosis factor alpha, and granulocyte-macrophage colony stimulating factor, wherein said antigen-presenting cells comprise one or more subsets selected from the group consisting of Langerhans cells with surface markers (CD1a+ CD14- CD207+); interstitial dendritic cells with surface markers (CD1a+ CD14+ CD207-); double-negative dendritic cells (DN-DCs) with surface markers (CD1a- CD14-); and dendritic cells with surface markers (CD1a- CD14+ CD209+), to manufacture a vaccine for inducing an immune response in a patient,
wherein said patient's cells upon contact with said loaded antigen-presenting cells in said vaccine mature to form cells expressing immunologic activity against said antigen, wherein said antigen loaded antigen-presenting cells are made in less than four days.

10. Use of antigen loaded antigen-presenting cells made by contacting monocytes *ex vivo* with tumor necrosis factor alpha and granulocyte-macrophage colony stimulating factor at one time point to form antigen-presenting cells and contacting said antigen-presenting cells with soluble or particulate antigenic material possessing at least one antigen for which an immune response is desired at a second time point to form antigen loaded antigen-presenting cells, wherein said antigen loaded antigen-presenting cells are produced in less than four days, and wherein said antigen-presenting cells comprise one or more subsets selected from the group consisting of Langerhans cells with surface markers (CD1a+ CD14- CD207+); interstitial dendritic cells with surface markers (CD1a+ CD14+ CD207-); double-negative dendritic cells (DN-DCs) with surface markers (CD1a- CD14-); and dendritic cells with surface markers (CD1a-CD14+ CD209+), to manufacture a vaccine for inducing an immune response in a patient,
wherein said patient's cells upon contact with said loaded antigen-presenting cells in said vaccine mature to form cells expressing immunologic activity against said antigen.

11. Use of cytotoxic T cells to manufacture a medicament for mounting an immune response in a patient, wherein said cytotoxic cells are produced by a method comprising:
producing antigen loaded antigen-presenting cells from monocytes *ex vivo* by simultaneously contacting said monocytes with soluble or particulate antigenic material possessing at least one antigen for which an immune response is desired, tumor necrosis factor alpha, and granulocyte-macrophage colony stimulating factor, wherein said antigen-presenting cells comprise one or more subsets selected from the group consisting of Langerhans cells with surface markers (CD1a+ CD 14- CD207+); interstitial dendritic cells with surface markers (CD1a+ CD14+ CD207-); double-negative dendritic cells (DN-DCs) with surface markers (CD1a- CD14-); and dendritic cells with surface markers (CD1a- CD14+ CD209+);
coculturing said loaded antigen-presenting cells with naïve T cells under conditions for T cell maturation to form cytotoxic T cells,
wherein said cytotoxic T cells express cytotoxic activity against target cells possessing said antigen, wherein said antigen loaded antigen-presenting cells are made in less than four days.

12. Use of cytotoxic T cells to manufacture a medicament for mounting an immune response in a patient, wherein said cytotoxic cells are produced by a method comprising:
producing antigen loaded antigen-presenting cells by contacting monocytes *ex vivo* with tumor necrosis factor alpha and granulocyte-macrophage colony stimulating factor at one time point to form antigen-presenting cells and contacting said antigen-presenting cells with soluble or particulate antigenic material possessing at least one antigen for which an immune response is desired at a second time point to form antigen loaded antigen-presenting cells, wherein said antigen loaded antigen-presenting cells are produced in less than four days, wherein said antigen-presenting cells comprise one or more subsets selected from the group consisting of Langerhans cells with surface markers (CD1a+ CD14- CD207+); interstitial dendritic cells with surface markers (CD1a+ CD14+ CD207-); double-negative dendritic cells (DN-DCs) with surface markers (CD1a- CD14-); and dendritic cells with surface markers (CD1a-CD14+ CD209+);
coculturing said loaded antigen-presenting cells with naive T cells under conditions for T cell maturation to form cytotoxic T cells,
wherein said cytotoxic T cells express cytotoxic activity against target cells possessing said antigen.

13. The use of claims 5, 6, 7, 8, 11, or 12, wherein cytotoxic cells are CD4 positive.

14. The use of claims 5, 6, 7, 8, 11, or 12, wherein cytotoxic cells are CD8 positive.

15. Use of antigen loaded antigen-presenting cells derived from monocytes *ex vivo* by simultaneously contacting said monocytes with soluble or particulate antigenic material possessing at least one antigen for which an immune response is desired, tumor necrosis factor alpha, and granulocyte-macrophage colony stimulating factor, wherein said antigen-presenting cells comprise one or more subsets selected from the group consisting of Langerhans cells with surface markers (CD1a+ CD14- CD207+); interstitial dendritic cells with surface markers (CD1a+ CD14+ CD207-); double-negative dendritic cells (DN-DCs) with surface markers (CD1a- CD14-); and dendritic cells with surface markers (CD1a- CD14+ CD209+), to manufacture a medicament for modifying an immune response in a patient,
wherein said patient's cells upon contact with said loaded antigen-presenting cells are modulated not to express immunologic activity against said antigen.
